(19) Europäisches Patentamt
European Patent Office
Office européen des brevets

(11) Publication number: 0 544 958 A1

# (12) EUROPEAN PATENT APPLICATION

(21) Application number: 91311373.4

(22) Date of filing: 06.12.91

(51) Int. Cl.[5]: C07D 501/20, C07D 417/12, C07D 215/24, A61K 31/545

(43) Date of publication of application: 09.06.93 Bulletin 93/23

(84) Designated Contracting States: AT BE CH DE DK ES FR GB GR IT LI LU NL SE

(71) Applicant: TANABE SEIYAKU CO., LTD.
2-10, Dosho-machi 3-chome
Chuo-ku Osaka(JP)

(72) Inventor: Saito, Kunio
No. 100-1, Dote-cho 2-chome
Omiya-shi, Saitama-ken(JP)
Inventor: Matsushita, Tadahiro
No. 27-6, Koganehara 1-chome
Matsudo-shi, Chiba-ken(JP)
Inventor: Takamura, Norio
No. 20-6, Shinshiraoka 2-chome,
Shiraoka-machi
Minamisaitama-gun, Saitama-ken(JP)
Inventor: Yamaguchi, Toutaro
No. 4-7, Ryoke 7-chome
Urawa-shi, Saitama-ken(JP)

(74) Representative: Baverstock, Michael George Douglas et al
BOULT, WADE & TENNANT 27 Furnival Street
London, EC4A 1PO (GB)

## (54) Cephalosporin compounds and processes for preparing the same.

(57) Cephalosporin compounds of the formula [I]:

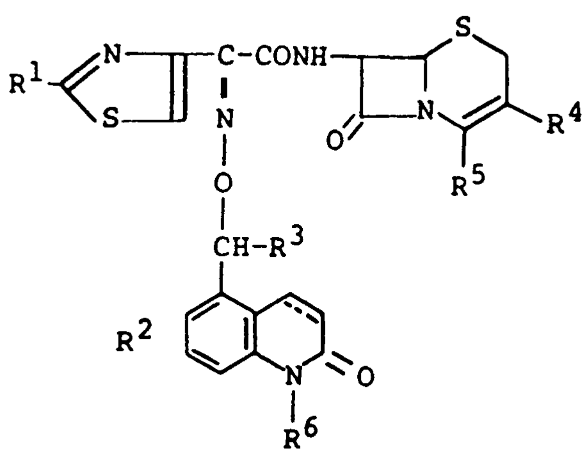

[I]

wherein $R^1$ is an amino or a protected amino group, $R^2$ is a hydroxy group, a protected hydroxy group or a lower alkoxy group, $R^3$ is a carboxyl group or a protected carboxyl group, $R^4$ is a hydrogen atom, a lower alkyl group, a lower alkenyl group or a group of the formula: $-CH_2R^{41}$, where $R^{41}$ is a nucleophilic residue, $R^5$ is a carboxyl group, a protected carboxyl group or a group of the formula -COO-, $R^6$ is a hydrogen atom or a lower alkyl group, and the broken line means the presence or absence of a double bond, or a pharmaceutically acceptable salt thereof, have excellent antimicrobial activities and are useful as antimicrobial drugs.

EP 0 544 958 A1

The present invention relates to novel cephalosporin compounds having excellent antimicrobial activities, processes for preparing the same and intermediates therefor.

Hitherto, many cephalosporin antibiotics have been known as antimicrobial agents, for example, it has been reported that (6R, 7R)-7-[(Z)-2-(2-aminothiazol-4-yl)-2-((2-carboxyprop-2-yl)oxyimino)acetamido]-3-(1-pyridiniomethyl)ceph-3-em-4-carboxylate (general name: ceftazidime) has potent antimicrobial activities [See Japanese Patent Second Publication (Kokoku) No. 5916/1987].

An object of the present invention is to provide novel cephalosporin compounds having excellent antimicrobial activities. Another object of the invention is to provide processed for preparing the novel cephalosporin compounds. A further object of the invention is to provide intermediates for preparing the novel cephalosporin compounds. These and other objects and advantages of the invention will be apparent to those skilled in the art from the following description.

The cephalosporin compounds of the present invention have the following formula [I]:

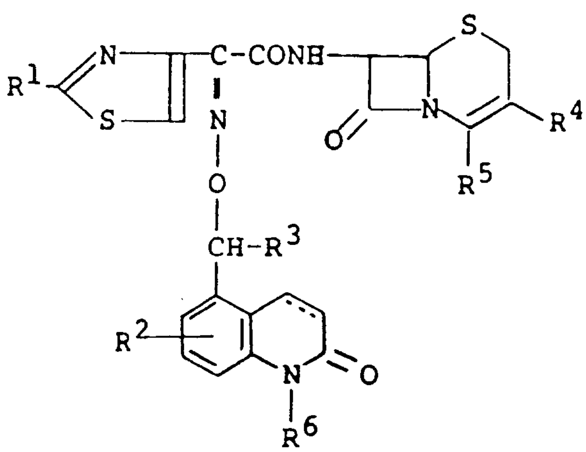

[I]

wherein $R^1$ is an amino group or a protected amino group, $R^2$ is a hydroxy group, a protected hydroxy group or a lower alkoxy group, $R^3$ is a carboxyl group or a protected carboxyl group $R^4$ is a hydrogen atom, a lower alkyl group, a lower alkenyl group or a group of the formula: $-CH_2R^{41}$, where $R^{41}$ is a nucleophilic residue, $R^5$ is a carboxy group, a protected carboxyl group or a group of the formula: $-COO^-$, $R^6$ is hydrogen atom or a lower alkyl group and the broken line means the presence or absence of a double bond.

The novel cephalosporin compounds [I] of the present invention and pharmaceutically acceptable salts thereof have excellent antimicrobial activities against a wide range of microorganisms including Gram positive bacteria and Gram negative bacteria, especially against Gram positive bacteria. The cephalosporin compounds [I] of the present invention also shod high stability to various $\beta$-lactamase-producing bacteria, and show high absorbability in living tissues, and further the effects of treatment last for a long time. In view of these advantages, the compounds [I] of the present invention can be used as antimicrobial agents, for example, in prophylaxis and treatment of infectious diseases caused by the above microorganisms, as a chemotherapeutic drug for mammals including human beings and also an additive for animal feeds.

The preferred compounds of the present invention include, for example, the compounds of the formula [I], wherein $R^4$ is a hydrogen atom, a lower alkyl group such as methyl, a lower alkenyl group such as vinyl, or a group of the formula: $-CH_2R^{41}$, wherein $R^{41}$ is a conventional nucleophilic residue used in this field, for example, a lower alkanoyloxy group such as acetoxy; a nitrogen-containing heterocyclic group such as a pyridinio group, quinolinio group, isoquinolinio group, 2,3-propanopyridinio group, 5,6,7,8-tetrahydroquinolinio group or 2,3-pentanopyridinio group. These heterocyclic groups may optionally have one or two substituents selected from a halogen atom (e.g. chlorine, bromine or fluorine), a lower alkyl group (e.g. methyl), a lower alkoxy group (e.g. methoxy), a lower alkoxycarbonyl group (e.g. methoxycarbonyl), amino group, carbamoyl group, cyano group, formylamino group and a hydroxy(lower)alkyl group (e.g. hydroxymethyl); $R^{41}$ may also be a thio group substituted by a nitrogen-containing heterocyclic group such as a thiadiazolylthio group, tetrazolylthio group or pyridiniothio group. These nitrogen-containing heterocyclic groups may optionally have a substituent selected from a lower alkyl (e.g. methyl) or lower alkenyl (e.g. vinyl or allyl).

Furthermore, compounds [I] of the present invention include, for example, compounds of formula [I] wherein $R^1$ is an amino group, $R^3$ is a carboxyl group, $R^5$ is a carboxyl group or a group of the formula:

-COO⁻, and $R^6$ is a hydrogen atom or methyl group.

Moreover, the compounds [I] of the present invention include, for example, compounds of formula [I], wherein $R^2$ is a hydroxy group or methoxy group which is substituted at the 8-position of the 2-oxo-1H-quinoline ring or the 2-oxo-1,2,3,4-tetrahydroquinoline ring.

The more preferred compounds of the invention as medicaments are compounds of formula [I], wherein $R^4$ is a group of the formula: $-CH_2R^{41}$, wherein $R^{41}$ is an acetoxy group, pyridinio group, 3-chloropyridinio group, 3-amino-2-methylpyridinio group or quinolinio group.

The most preferred compounds of the invention as medicaments are compounds of formula [I], wherein $R^4$ is a group of the formula: $-CH_2R^{41}$ wherein $R^{41}$ is a pyridinio group, 3-chloropyridinio group, 3-amino-2-methylpyridinio group or quinolinio group.

When $R^1$ in compound [I] of the present invention is a protected amino group, the protecting group for the amino group includes various conventional protecting groups which are usually used in the synthesis of peptides, for example, a loner alkanoyl group; mono-, di- or trihalogeno(lower)alkanoyl group; a lower alkoxycarbonyl group; a substituted or unsubstituted phenyl(lower)alkoxycarbonyl group such as a benzyloxycarbonyl group, p-(lower)alkoxybenzyloxycarbonyl group; a substituted or unsubstituted phenyl-(lower)alkyl group much as a benzyl group, p-(lower)alkoxybenzyl group, 3,4-di(lower)alkoxybenzyl group; and di- or triphenyl(lower)alkyl groups.

When $R^2$ of compound [I] of the present invention is a protected hydroxy group, the protecting group for the hydroxy group includes, for example, a substituted or unsubstituted phenyl(lower)alkyl group such as benzyl, or diphenylmethyl; a lower alkanoyl group such as an acetyl group or benzoyl group.

Moreover, when $R^3$ and/or $R^5$ of compound [I] of the present invention are protected carboxyl groups, the protecting group for the carboxyl group is preferably a protecting group which can be easily removed from the carboxyl group by a conventional method such as hydrolysis, acid-treatment or reduction. Such protecting groups are, for example, a lower alkyl group; a substituted or unsubstituted phenyl(lower)alkyl group such as benzyl, p-(lower)alkoxybenzyl, p-nitrobenzyl, or diphenylmethyl; and a tri(lower)alkylsilyl group.

According to the present invention, the desired compounds [I] and pharmaceutically acceptable salts thereof can be prepared by condensing an oxyiminoacetic acid compound of the formula [II]:

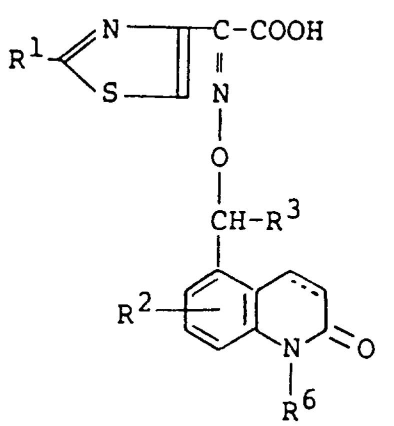

[II]

wherein the symbols are the same as defined above, a salt or a reactive derivative thereof, with a 7-aminocephalosporin compound of the formula [III]:

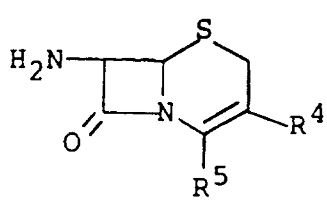

[III]

wherein the symbols are the same as defined above, or a salt thereof, or by reacting an oxyiminocephaloporin compound of the formula [IV]:

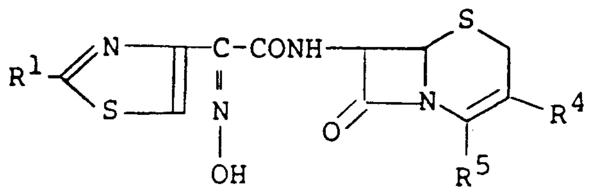

[IV]

wherein the symbols are the same as defined above, or a salt thereof, with a carbostyril compound of the formula [V]:

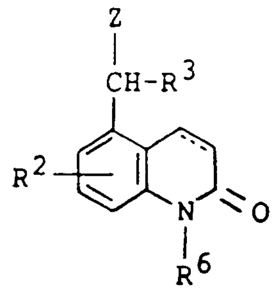

[V]

wherein Z is a reactive residue, $R^2$, $R^3$, $R^6$ and the broken line are the same as defined above, or a salt thereof.

Among the desired compounds [I] of the present invention, the compound wherein $R^4$ is a group of the formula: $-CH_2R^{41}$ can be prepared by reacting a cephalosporin compound of the formula [VI]:

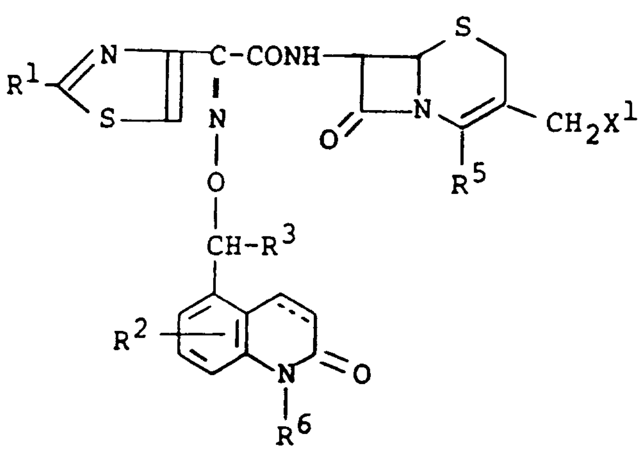

[VI]

wherein $X^1$ is a reactive residue, $R^1$, $R^2$, $R^3$, $R^5$, $R^6$ and the line are the same as defined above, or a salt thereof, with a nucleophilic compound [VII] or a salt thereof.

When the desired compounds [I] thus prepared are the compounds wherein $R^1$ is a protected amino group, $R^2$ is a protected hydroxy group, and/or $R^3$ and/or $R^5$ are protected carboxyl groups, if desired the said protecting groups may optionally be removed. Additionally, the desired compounds [I] may optionally be converted into pharmaceutically acceptable salts thereof.

The salts for the starting compounds [II], [III], [IV], [V] and [VI] may preferably be any salt with a conventional inorganic or organic amine.

The starting nucleophilic compound [VII] includes any conventional compounds which are usually used in this field, for example, a lower alkane acid (e.g. acetic acid) a nitrogen-containing heterocyclic compound (e.g. pyridine, quinoline, isoquinoline, 2,3-propanopyridine, tetrahydroquinoline or 2,3-pentanopyridine) which may optionally have substituents selected from the group consisting of a halogen atom, a lower alkyl, a lower alkoxy, a lower alkoxycarbonyl, amino, carbamoyl, cyano, formylamino and a hydroxy(lower)alkyl, and a mercapto- or thiosubsituted nitrogen-containing heterocyclic compound (e.g. mercaptothiadiazole, mercaptotetrazole or thiopyridone) which may optionally have a substituent selected from the group

consisting of a lower alkyl and a lower alkenyl. These nucleophilic compounds [VII] may be used in the form of salts such as mineral acid salts or alkali metal salts.

The condensation reaction between the free oxyiminoacetic acid [II] and the 7-aminocephalosporin compound [III] may be carried out in the presence of a dehydrating agent. The dehydrating agent may be any conventional dehydrating agent, for example, dicyclohexylcarbodiimide, phosphorus oxychloride, thionyl chloride, oxalic chloride or a Vilsmeier reagent which is prepared by reacting dimethylformamide with phosphorus oxychloride, oxalic chloride, phosgene or thionyl chloride. This reaction is preferably carried out under cooling or at room temperature.

The condensation reaction between a reactive derivative (e.g. acid anhydride, mixed acid anhydride, or active ester) of the oxyiminoacetic acid compound [II] and the 7-aminocephalosporin compound [III] or a salt thereof may be carried out in the presence or absence of a base. The base may preferably include, for example, an alkali metal hydroxide, an alkali carbonate, an alkali hydrogen carbonate, bis(trimethylsilyl)-acetamide, trialkylamine, N,N-dialkylaniline or pyridine. This reaction is preferably carried out under cooling or at room temperature (e.g. -60 ° C to 25 ° C).

The reaction between the oxyiminocephalosporin compound [IV] and the carbostyril compound [V] is carried out in the presence of an dehydrating agent or a base. The reactive reside for Z of the carbostyril compound [V] is, for example, a hydroxy group, a halogen atom or a lower alkylsulfonyloxy group. The dehydrating agent may be, for example, a mixture of a phosphorus compound (e.g. triphenylphosphine, diphenylmethylphosphine, or diphenylethylphosphine), and an azodicarboxylic acid di(lower)alkyl ester. The base is preferably an alkali metal hydroxide, a trialkylamine or N,N-dialkylaniline, pyridine. This reaction is preferably carried out under cooling or with warming (e.g. -40 ° C to 30 ° C).

The reaction between the cephalosporin compound [VI] and the nucleophilic compound [VII] is preferably carried out in a suitable solvent. In this reaction, the cephalosporin compound [VI] may be a compound of the formula [VI] wherein the reactive residue for $X^1$ is, for example, a carbamoyloxy group, a lower alkanoyloxy group or a halogen atom. This reaction is preferably carried out at room temperature, under cooling or with heating (e.g. -40 ° C to 40 ° C), at pH 2 - 8, more preferably at pH 5 - 8. If desired, in order to promote the reaction, there may be added to the reaction system an alkali metal halide, a tri(lower)-alkylsilyltrifluoromethanesulfonate, an alkali metal thiocyanate, an alkali metal hydrogen carbonate, a quaternary ammonium salt having surface activity [e.g. tri(lower)alkylbenzylammonium halide) or phosphate buffer solution.

The reactions mentioned above may, if desired, be carried out in a suitable solvent such as dimethylformamide, dimethylacetamide, dioxane, acetone, alcohol, acetonitril, methylene chloride, chloroform, pyridine, dimethylsulfoxide, toluene, tetrahydrofuran, water or a mixture thereof.

In the above reactions, optical active compounds [I] of the present invention may be obtained from optical active starting compounds [II], [V] or [VI].

The compounds [I] wherein $R^1$ is a protected amino group, $R^2$ is a protected hydroxy group, and/or $R^3$ and/or $R^5$ are a protected carboxyl group, may optionally be subjected to removal of the protecting groups. The removal of these protecting groups is carried out in a conventional manner, for example, hydrolysis, solvolysis, acid-treatment, or reduction, or a combination thereof, which is selected depending on the kind of protecting groups to be removed.

The compounds [I] of the present invention and pharmaceutically acceptable salts thereof have excellent antimicrobial activities against the wide range of Gram positive bacteria, such as microorganisms of the genera Enterococcus (e.g. Enterococcus faecalis), Staphylococcus (e.g. Staphylococcus aureus, Staphylococcus epidermidis), Morganella (e.g. Morganella morganii) and Pseudomonas(e.g. Pseudomonas aeruginosa) and Gram negative bacteria. In particular, unlike the above mentioned conventional cephalosporin compounds, the compounds [I] of the present invention and pharmaceutically acceptable salts thereof have potent antimicrobial activities against Gram positive bacteria (e.g. microorganisms of Staphylococcus).

For example, as compared with the above mentioned ceftazidime, the compound of this invention, 7β-{(Z)-2-(2-aminothiazol-4-yl)-2-[(8-hydroxy-2-oxo-1H-quinolin-5-yl)(carboxy)methyloxyimino]acetamido}-3-(1-quinoliniomethyl)-3-cephem-4-carboxylic acid has excellent antimicrobial activity, more than 32 times stronger against Staphylococcus aureus 209P JC-1, and 7β-{(Z)-2-(2-aminothiazol-4-yl)-2-[(8-hydroxy-2-oxo-1H-quinolin-5-yl)(carboxy)methyloxyimino]acetamido}-3-(1-pyridiniomethyl)-3-cephem-4-carboxylicacid and 7β-{(Z)-2-(2-aminothiazol-4-yl)-2-[(8-hydroxy-2-oxo-1H-quinolin-5-yl)(carboxy)methyloxyimino]-acetamido}-3-(3-amino-2-methyl-1-pyridiniomethyl)-3-cephem-4-carboxylic acid have excellent antimicrobial activity, more than 16 times stronger against Staphylococcus epidermidis Kawamura.

Moreover, the desired compounds [I] of the present invention and pharmaceutically acceptable salts thereof characteristically have higher stability to various β-lactamase-producing bacteria. For example, the

compounds of the present invention, 3-(1-quinoliniomethyl) derivative and 3-(1-pyridiniomethyl) derivative of 7β-{(Z)-2-(2-aminothiazol-4-yl)-2-[(8-hydroxy-2-oxo-1H-quinolin-5-yl)(carboxy)methyloxyimino]acetamido}-3-cephem-4-carboxylic acid have excellent antimicrobial activity, more than 4 times stronger against Citrobacter freundii GN346 than ceftazidime.

The desired compounds [I] of the present invention and pharmaceutically acceptable salts thereof also have excellent antimicrobial activities against microorganisms of the genera Proteus (e.g. Proteus morganii), Citrobacter (e.g. Citrobacter freundii), Escherichia (e.g. Escherichia coli), Klebsiella (e.g. Klebsiella pneumoniae), and Enterobacter, Serratia, Shigella and Salmonella.

The most important characteristic of these compounds is to show potent effect on clinical isolates of methicillin resistant Staphylococcus aureus (MRSA) which is resistant to many β-lactam antibiotics. For example, antibacterial activity (90 % inhibitory concentration) of 7β-{((Z)-2-(2-aminothiazol-4-yl)-2-[(8-hydroxy-2-oxo-1H-quinolin-5-yl)(carboxy)methyloxyimino]acetamido}-3-(3-amino-2-methyl-1-pyridiniomethyl)-3-cephem-4-carboxylic acid against clinical isolates of MRSA is about 16 times superior to that of ceftazidime. Further, antibacterial activity (90 % inhibitory concentration) of the above-mentioned compound of the invention against clinical isolates of P. aeruginosa is about twice as superior to ceftazidime.

Moreover, the desired compounds [I] or pharmaceutically acceptable salts thereof have high absorbability in the living tissue, and the treatment effects thereof last for a long time so that the compounds [I] of the present invention show an excellent defense effect in infectious diseases caused by various microorganisms including Staphylococcus aureus and Pseudomonas aeruginosa. In addition, the desired compounds [I] of the present invention and pharmaceutically acceptable salts thereof have low toxicity and show high safety as medicaments. For example, when acute toxicity of 7β-{(z)-2-(2-aminothiazol-4-yl)-2-[(8-hydroxy-2-oxo-1H-quinolin-5-yl) (carboxy)methyloxyimino]acetamido}-3-(3-amino-2-methyl-1-pyridiniomethyl)-3-cephem-4-carboxylic acid was tested by intravenous administration using mice, none of the five mice which were administered at a dose of 1 g/kg died within five days after administration.

The cephalosporin compounds [I] of the present invention may be used either in the free form or in the form of pharmaceutically acceptable salts thereof. The pharmaceutically acceptable salts include, for example, non-toxic metal salts such as the sodium salt, potassium salt, calcium salt, magnesium salt, and aluminum salt; salts with non-toxic amines such as trialkylamine (e.g. triethylamine), pyridine, ethanolamine, triethanolamine, and dicyclohexylamine; salts with inorganic acids such as hydrochloric acid, sulfuric acid and hydrobromic acid; salts with organic acids such as oxalic acid and tartaric acid; addition salts with amino acids such as glycine, lysine, arginine, aspartic acid and glutamic acid.

The salt of compound [I] may also be a salt with a resin such as a polystyrene resin having an amino group, quaternary amino group or sulfonic acid group, or a resin having a carboxyl group such as a resin comprising a polyacrylic acid resin. The compounds [I] of the present invention may also be in the form of a complex with a metal such as iron or copper, or with an ammonium salt such as ammonium chloride. Thus, the desired compounds [I] of the present invention and salts thereof include inner salts, addition salts, complexes, solvates and hydrates thereof.

The cephalosporin compounds [I] or pharmaceutically acceptable salts thereof can be administered either orally or parenterally (e.g. by the intravenous, intramuscular or subcutaneous route). They can be used in the form of a pharmaceutical preparation suitable for oral or parenteral administration in admixture with a pharmaceutically acceptable carrier or diluent, for example, tablets, granules, capsules, powder or injections.

The daily dose of the compounds [I] of the present invention may vary depending on administration route, age, weight and condition of the patient, and kind of the disease to be cured, but it is usually in the range of about 2 to 200 mg/kg of body weight, preferably about 5 to 40 mg/kg of body weight.

In this specification and claims, the partial structure of the formula:

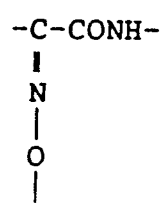

means, if not defined otherwise, the geometrical isomers of the following formulae:

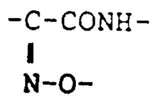

or

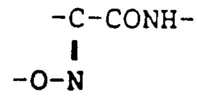

(Z)-isomer (E)-isomer

or a mixture thereof. However, the compounds [I] or pharmaceutically acceptable salts thereof wherein the oxyimino group has the (Z)-configuration are preferably used as a medicament, and show superior biological activity.

The desired compounds [I] of the present invention, the starting compounds [II] and [VI] include either optical isomers or a racemic mixture owing to the partial structure of the following formula:

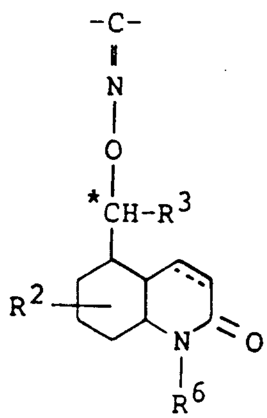

wherein the symbol * means an asymmetric carbon atom, $R^2$, $R^3$, $R^6$ and the broken line are as defined above.

The starting compound [II] is a novel compound, and can be prepared, for example, by reacting the carbostyril compound [V] with a compound of the formula [VIII]:

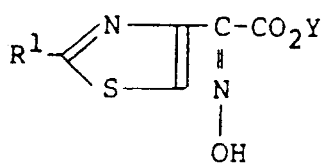

[VIII]

wherein the group $-CO_2Y$ is carboxyl group or a protected carboxyl group, $R^1$ is the same as defined above, in the presence of a base under cooling or with heating to give a compound of the formula [II-a]:

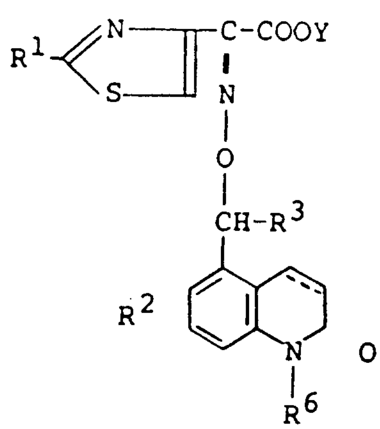

[II-a]

wherein the symbols are the same as defined above, optionally followed by removal of the protecting group from the compound [II-a]. When a group of the formula: $-CO_2Y$ is a protected carboxyl, the protecting group therefor may be as exemplified above for the protecting group for $R^3$ and $R^5$. Alternatively, the compound [II] may also be prepared by reacting the carbostyril compound [V] with N-hydroxyphthalimide or an alkali metal salt thereof, followed by treating the reaction mixture with a lower alkylhydrazine under cooling to give a compound of the formula [IX]:

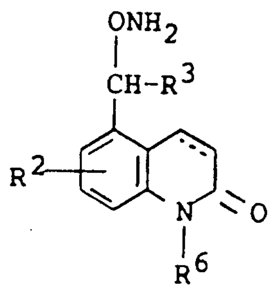

[IX]

wherein the symbols are the same as defined above, and then reacting the compound [IX] with a glyoxylic acid compound of the formula [X]:

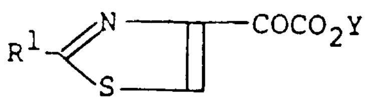

[X]

wherein the symbols are the same as defined above, in the presence or absence of a base under cooling or with heating, optionally followed by removal of the protecting group.

Throughout the description and claims, the lower alkyl, lower alkenyl, lower alkoxyl and lower alkanoyl groups have 1 to 6 carbon atoms, 2 to 6 carbon atoms, 1 to 6 carbon atoms and 2 to 6 carbon atoms, respectively.

The present invention is illustrated in more detail by the following Examples and Reference Examples, but should not be construed to be limited thereto.

Example 1

(1)-(a) To a mixture of 2-(8-diphenylmethyloxy-2-oxo-1H-quinolin-5-yl)-2-hydroxyactic acid diphenylmethyl ester (47.7 g), N-hydroxyphthalimide (41.2 g), triphenylphosphine (55.2 g) and tetrahydrofuran (2000 ml) is added dropwise azodicarboxylic acid diethyl ester (36.6 g) under argon atmosphere at 5 to 10 °C. The mixture is stirred at the same temperature for one hour, and further at room temperature for 70 hours. The mixture is concentrated, and the resulting residue is purified by silica gel column chromatography (eluent; hexane/ethyl acetate and chloroform), recrystallized from chloroform/ether to give 2-(8-diphenylmethyloxy-2-oxo-1H-quinolin-5-yl)-2-(phthalimidoxy)acetic acid diphenylmethyl ester (44.5 g).

M.p. 206 - 207 °C (decomposed)

IR (Nujol) : 3170, 3130, 3065, 3030, 1790, 1760, 1730, 1660, 1610 $cm^{-1}$

MS (m/z): 713 ($MH^+$)

(1)-(b) A suspension of 2-(8-diphenylmethyloxy-2-oxo-1H-quinolin-5-yl)-2-hydroxyacetic acid diphenylmethyl ester (0.57 g) and calcium carbonate (0.2 g) in tetrahydrofuran (10 ml) is cooled to -5 to 0 °C and stirred under argon atmosphere. To the mixture is added dropwise phosphorus tribromide (0.38 g), and the mixture is further stirred for one hour. The mixture is poured into a cold mixture of ethyl acetate (10 ml) and 2N-aqueous sodium hydroxide solution (3 ml) with stirring at a temperature below 2 °C, and basified with saturated aqueous sodium hydrogen carbonate solution, and separated. The aqueous layer is extracted with ethyl acetate, and the extract is combined with the above obtained ethyl acetate layer, washed with saline solution, dried, and thereto is added calcium carbonate (5 mg). The mixture is evaporated to remove the solvent to give 2-bromo-2-(8-diphenylmethyloxy-2-oxo-1H-quinolin-5-yl)acetic acid diphenylmethyl ester.

A suspension of N-hydroxyphthalimide potassium salt (0.25 g) in dimethylformamide (2 ml) is cooled to -10 °C under argon atmosphere, and thereto is added dropwise a solution of the above compound in dimethylformamide (3 ml), and the mixture is stirred at 0 to 2 °C for one hour. The mixture is poured into a mixture of ice-water (20 ml)/ethyl acetate (30 ml), and separated. The organic layer is collected, washed with aqueous sodium hydrogen carbonate solution and water, dried and concentrated. The resulting residue is purified by silica gel column chromatography to give 2-(8-diphenylmethyloxy-2-oxo-1H-quinolin-5-yl)-2-(phthalimidoxy)acetic acid diphenylmethyl ester (0.30 g). The physicochemical properties of this product are the same as those of the preparation obtained in above step (a).

(2) A solution of the product (36.5 g) obtained above in methylene chloride (500 ml) is cooled to -60 °C under argon atmosphere, and thereto is added dropwise methylhydrazine (3.0 ml). The mixture is stirred at -60 to 0 °C for 1.5 hour, and further at 0 °C for 30 minutes. The precipitated crystals are removed by filtration, and washed with methylene chloride. The washings and the filtrate are combined, and evaporated. The residue is crystallized from ether and the precipitated crystals are collected by filtration, washed with ether and dried to give 2-aminooxy-2-(8-diphenylmethyloxy-2-oxo-1H-quinolin-5-yl)acetic acid diphenylmethyl ester (29.8 g) as colorless crystals.

M.p. 178 - 180 °C (decomposed)

IR (Nujol): 3230, 3160, 1750, 1730, 1650, 1600 $cm^{-1}$

MS (m/z): 583 ($MH^+$)

This product is recrystallized from methanol to give crystals having the different IR spectrum.

M.p. 170 - 171 °C

IR (Nujol): 3650, 3550, 3300, 3240, 1760, 1740, 1660, 1610 $cm^{-1}$

(3) The product (28 g) obtained above and 2-tritylaminothiazol-4-yl-glyoxylicacid (18.65 g) are dissolved in a mixture of tetrahydrofuran (800 ml), acetonitril (280 ml) and water (280 ml), and the mixture is stirred at room temperature for five hours, and evaporated to remove the solvent. The residue is crystallized from ether and the precipitated crystals are collected by filtration, washed with ether, and dried to give 2-(2-tritylaminothiazol-4-yl)-2[(8-diphenylmethyloxy-2-oxo-1H-quinolin-5-yl)(diphenylmethyloxycarbonyl)-methyloxyimino]acetic acid (41.6 g) as colorless crystals.

M.p. 154 - 156 °C (decomposed)

IR (Nujol): 3400, 3200, 3060, 3030, 2800 - 2400, 1740, 1720, 1660, 1610 $cm^{-1}$

Mz (m/z): 979 ($MH^+$)

(4) To dimethylformamide (2.23 g) are added successively phosphorus oxychloride (2.85 g) and methylene chloride (10 ml) with ice-cooling under argon atmosphere, and the mixture is stirred at room temperature for one hour, and then cooled to -55 to -50 °C. To the mixture is added dropwise a solution of 2-(2-tritylaminothiazol-4-yl)-2-[(8-diphenylmethyloxy-2-oxo-1H-quinolin-5-yl)-(diphenylmethyloxycarbonyl)methyloxyimino)acetic acid (13 g) in methylene chloride (300 ml), and the mixture is stirred at -60 to -50 °C for two hours (the resulting solution is referred to as Reaction Solution A). Alternatively, bis(trimethylsilyl)acetamide (14.8 ml) is added to a suspension of 7-aminocephalosporanic acid (5.43 g) in methylene chloride (48 ml) with ice-cooling under argon atmosphere, and the mixture is stirred at the same temperature for 1.5 hour (the resulting solution is referred to as Reaction Solution B). Reaction Solution B is added dropwise to Reaction Solution A at -60 to -55 °C, and the mixture is stirred at -60 to -30 °C for one hour, then at -30 °C for 1.5 hour. After completion of the reaction, the mixture is poured into ice-water and separated. The aqueous layer is extracted with methylene chloride, and the extract is combined with the methylene chloride layer, washed with saline solution, dried and evaporated to remove the solvent to give 70-{(Z)-2-(2-tritylaminothiazol-4-yl)-2-[(8-diphenylmethyloxy-2-oxo-1H-quinolin-5-yl)(diphenylmethyloxycarbonyl)-methyloxyimino]acetamido}cephalosporanic acid (16.48 g) as light yellow powder.

M.p. 160 - 162 °C (decomposed)

IR (Nujol): 3380, 3230, 2800 - 2400, 1790, 1740, 1670, 1610 $cm^{-1}$

MS (m/z): 1233 ($MH^+$)

## Example 2

7β-{(Z)-2-(2-Tritylaminothiazol-4-yl)-2-[(8-diphenylmethyloxy-2-oxo-1H-quinolin-5-yl)-(diphenylmethyloxycarbonyl)methyloxyimino]acetamido}cephalosporanic acid (12.48 g) is dissolved in formic acid (160 ml), and thereto is added water (40 ml) under ice-cooling, and the mixture is stirred at room temperature for 8.5 hours. The precipitates are separated by filtration, and washed with 80 % aqueous formic acid solution. The filtrate and the washings are combined, and evaporated to remove the solvent. To the residue is added ethanol, and the precipitate powder is collected by filtration, washed with ethanol and

ether, dried and dissolved in aqueous sodium hydrogen carbonate solution. The pH value of the mixture is adjusted to about pH 8.0 with saturated aqueous sodium hydrogen carbonate solution, and purified with a column packed with a nonionic adsorbing resin (tradename: Diaion HP-20 manufactured by Mitsubishi Kasei Corporation, hereinafter referred to as HP-20) (eluent; water and 5 % aqueous methanol). The fractions containing the desired compound are combined, concentrated, and lyophilized to give 7β-{(Z)-2-(2-aminothiazol-4-yl)-2[(8-hydroxy-2-oxo-1H-quinolin-5-yl)(carboxy)methyloxyimino)acetamido}cephalosporanic acid disodium salt (4.2 g) as light yellow powder.

M.p. 230 - 240 °C (decomposed)

IR (Nujol): 3320, 3200, 1770, 1730, 1650, 1600 $cm^{-1}$

MS (m/z): 703 ($MH^+$)

NMR ($D_2O$) δ: 2.11 and 2.12 (3H, sx2), 2.88 and 2.92 (1H, dx2, J = 18Hz and 18Hz), 3.36 and 3.39 (1H, dx2, J = 18Hz and 18Hz), 4.66 (1H, d, J = 12Hz), 4.83 (1H, d, J = 12Hz), 4.89 and 4.92 (1H, dx2, J = 4.9Hz and 4.4Hz), 5.56 and 5.66 (1H, dx2, J = 4.9Hz and 4.4Hz), 5.97 (1H, s), 6.75 and 6.76 (1H, dx2, J = 9.8Hz and 9.8Hz), 6.99-7.05 (2H, m), 7.21 and 7.23 (1H, dx2, J = 8. 3Hz and 7.8Hz), 8.28 and 8.35 (1H, dx2, J = 9. 8Hz and 9.8 Hz)

Example 3

(1) To a solution of quinoline (900 mg) in methylene chloride (9 ml) is added trimethylsilyltrifluoromethanesulfonate (1.1 ml) under argon atmosphere, and the mixture is stirred at room temperature for 10 minutes. To the mixture is added 7β-{(Z)-2-(2-tritylaminothiazol-4-yl)-2-[(8-diphenylmethyloxy-2-oxo-1H-quinolin-5-yl)(diphenylmethyloxycarbonyl)methyloxyimino]acetamido}cephalosporanic acid (1 g), and the mixture is stirred at room temperature for 45 minutes, and then refluxed with stirring for 1.5 hours. The solvent is distilled off, and the resulting residue is dissolved in formic acid (12 ml), and thereto is added water. The mixture is stirred at room temperature for 6.5 hours. The precipitates are separated by filtration, washed with 80 % aqueous formic acid solution, and the washings and the filtrate are combined. The solvent is distilled off, and the residue is dissolved in saturated aqueous sodium hydrogen carbonate solution and acetonitril, and the pH value thereof is adjusted to about pH 8. Acetonitril is distilled off from the mixture, and the aqueous layer is washed with chloroform, and purified by HP-20 column chromatography (eluent; water and 5 - 30 % aqueous metanol). The fractions containing the desired compound are combined, concentrated, and lyophilized to give 7β-{(Z)-2-(2-aminothiazol-4-yl)-2-[(8-hydroxy-2-oxo-1H-quinolin-5-yl)(caboxy)methyloxyimino]acetamido}-3-(1-quinoliniomethyl)-3-cephem-4-carboxylic acid sodium salt (160 mg) as slightly yellow powder.

M.p. 205 - 210 °C (decomposed)

IR (Nujol): 3350, 3200, 1770, 1660, 1610 $cm^{-1}$

MS (m/z): 750 ($MH^+$)

NMR ($D_2O$) δ: 2.58 and 2.65 (1H, dx2, J = 19Hz and 19Hz), 3.12 and 3.15 (1H, dx2, J = 19Hz and 19Hz), 4.91 and 4.94 (1H, dx2, J = 4.9Hz and 4.9Hz), 5.62 and 5.68 (1H, dx2, J = 4.9Hz and 4.9Hz), 5.70 and 5.85 (2H, each br d), 5.93 (1H, s), 6.60 and 6.64 (1H, dx2, J = 9.8Hz and 9.8Hz), 6.71 and 6.86 (1H, dx2, J = 8.3Hz and 8.3Hz), 6.92 and 6.94 (1H, sx2), 7.12 and 7.18 (1H, dx2, J = 8. 3Hz and 8. 3Hz), 7.80-8.34 (6H, m), 8.98-9.10 (2H, m)

(2) The product (100 mg) obtained above is subjected to high performance liquid chromatography to isolate each isomer, and further purified by HP-20 column chromatography to give a-isomer and a-isomer, separately.

Retention time (Rt) of a-isomer: 25 minutes

Retention time (Rt) of β-isomer: 40 minutes

[Conditions]

Solid phase; $C_{18}$-silica gel (tradename: YMC-Pack S-343)
Eluent; Acetonitril : 30 mM phosphate buffer (pH 7.0) = 8.5 : 91.5
Flow rate; 5 ml/minute

α-Isomer

Yield: 30 mg

M.p. >175 °C (decomposed)

IR (Nujol): 3380, 3200, 1770, 1660, 1610 $cm^{-1}$

MS (m/z): 750 ($MH^+$)

NMR ($D_2$0) δ: 2.62, 3.17 (2H, dx2, J = 18Hz and 18Hz), 4.94 (1H, d, J = 4.9Hz), 5.62 (1H, d, J = 4.9Hz), 5.75, 5.91 (2H, dx2, J = 16Hz and 16Hz), 5.95 (1H, s), 6.66 (1H, d, J = 9.8Hz), 6.79 (1H, d, J = 8.3Hz), 6.99 (1H, s), 7.13 (1H, d, J = 8.3Hz), 7.90-8.40 (6H, m), 9.10 (2H, m)

β-Isomer

Yield: 27 mg

M.p. >175 °C (decomposed)

IR (Nujol): 3380, 3200, 1770, 1660, 1610 $cm^{-1}$

MR (m/z): 750 ($MH^+$)

NMR ($D_2O$) δ: 2.57, 3.13 (2H, dx2, J = 18Hz and 18 Hz), 4.91 (1H, d, J = 4.9Hz), 5.69 (1H, d, J = 4.9Hz), 5.73, 5.88 (2H, dx2, J = 16Hz and 16Hz), 5.96 (1H, s), 6.68 (1H, d, J = 10Hz), 6.94 (1H, d, J = 8.3Hz), 6.98 (1H, s), 7.20 (1H, d, J = 8.3Hz), 7.80 - 8.38 (6H, m), 9.12 (2H, m)

Example 4

To a mixture of 7β-{(Z)-2-(2-aminothiazol-4-yl)-2-[(8-hydroxy-2-oxo-1H-quinolin-5-yl)(carboxy)-methyloxyimino]acetamido}cephalosporanic acid disodium salt (0.59 g), pyridine (0.34 ml) and sodium iodide (1.25 g) in water (6 ml) is added acetic acid in order to adjust the pH value of the mixture to pH 6.6. The mixture is stirred at 70 °C under argon atmosphere for 30 minutes, and then cooled. The pH value of the mixture is adjusted to pH 7.9 with saturated aqueous sodium hydrogen carbonate solution, and the mixture is purified by HP-20 column chromatography (eluent; water and 5 % aqueous methanol). The initially eluating part, the middle part and the last part of the fractions containing the desired compound are combined separately, and concentrated. The each resulting residue is lyophilized to give α-isomer, a mixture of α-isomer and β-isomer, and β-isomer of 7β-{(Z)-2-(2-aminothiazol-4-yl)-2-[(8-hydroxy-2-oxo-1H-quinolin-5-yl)(carboxy)methyloxyimino]acetamido}-3-(1-pyridiniomethyl)-3-cephem-4-carboxylic acid sodium salt.

α-Isomer

Yield: 24 mg

M.p. >200 °C (decomposed)

IR (Nujol): 3350, 3320, 1770, 1660 - 1620, 1600 $cm^{-1}$

MS (m/z): 722 ($MNa^+$), 700 ($MH^+$)

NMR ($D_2O$) δ: 2.61 (1H, d, J = 18Hz), 3.34 (1H, d, J = 18Hz), 4.96 (1H, d, J = 4.9Hz), 5.28 (1H, d, J = 14Hz), 5.49 (1H, d, J = 14Hz), 5.70 (1H, d, J = 4.9Hz), 5.96 (1H, s), 6.67 (1H, d, J = 9.8Hz), 6.90 (1H, d, J = 8.3Hz), 7.19 (1H, d, J = 8.3Hz), 8.13 (2H, m), 8.32 (1H, d, J = 9.8Hz), 8.60 (1H, m), 8.90 (2H, m)

β-Isomer

Yield: 16 mg

M.p. >200 °C (decomposed)

IR (Nujol): 3350, 3320, 1770, 1660 - 1620, 1600 $cm^{-1}$

MS (m/z): 722 ($MNa^+$), 700 ($MH^+$)

NMR ($D_2O$) δ: 2.64 (1H, d, J = 18Hz), 3.34 (1H, d, J = 18Hz), 4.97 (1H, d, J = 4.4Hz), 5.28 (1H, d, J = 15Hz), 5.51 (1H, d, J = 15Hz), 5.61 (1H, d, J = 4.4Hz), 5.96 (1H, s), 6.64 (1H, d, J = 8.3Hz), 6.69 (1H, d, J = 9.8Hz), 7.06 (1H, d, J = 8.3Hz), 8.13 (2H, m), 8.22 (1H, d, J = 9.8Hz), 8.60 (1H, m), 8.90 (2H, m)

A mixture of α- and β-isomers

Yield: 91 mg

M.p. >200 °C

IR (Nujol): 3350, 3200, 1770, 1660 - 1620, 1600 $cm^{-1}$

MS (m/z): 722 ($MNa^+$), 700 ($MH^+$)

NMR ($D_2O$) δ: 2.16 and 2.64 (1H, dx2, J = 18Hz and 18Hz), 3.34 (1H, d, J = 18Hz), 4.96 and 4.97 (1H, dx2, J = 4.9Hz and 4.4Hz), 5.28 (1H, br d), 5.49 and 5.51 (1H, dx2, J = 14Hz and 15Hz), 5.61 and 5.70 (1H, dx2, J = 4.4Hz and 4.9Hz), 5.96 (1H, br d), 6.66 and 6.90 (2H, m and d, J = 8.3Hz), 7.00 (1H, s), 7.06 and

7.19 (1H, dx2, J = 8.3Hz and 8.3Hz), 8.13 (2H, m), 8.22 and 8.32 (1H, dx2, J = 9.8Hz and 9.8Hz), 8.60 (1H, m), 8.90 (2H, m)

Examples 5 - 26

7β-{(Z)-2-(2-Tritylaminothiazol-4-yl)-2-[(8-diphenylmethyloxy-2-oxo-1H-quinolin-5-yl)-(diphenylmethyloxycarbonyl)methyloxyimino]acetamido}cephalosporanic acid and a nitrogen-containing heterocyclic compound are treated in the same manner as in Example 3 to give the compounds of the following Table 1.

Table 1

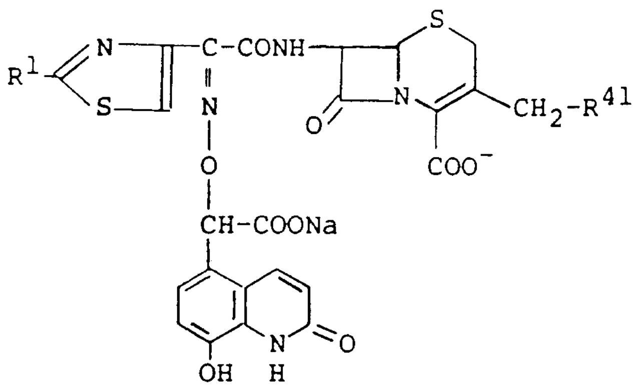

| Ex. No. | Compound | |
|---|---|---|
| | $R^{41}$ | Physicochemical properties |
| 5 | 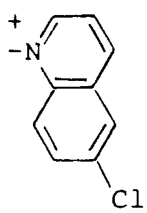 | M.p. 210-220°C (decomposed)<br>IR $\nu_{max}^{nujol}$ $cm^{-1}$: 3350, 3200, 1770, 1660, 1610<br>NMR ($D_2O$) δ: 2.58 and 2.66 (1H, dx2, J=18Hz and 18Hz), 3.19 and 3.22 (1H, dx2, J=18Hz and 18Hz), 4.96 (1H, d, J=4.9Hz), 5.63 and 5.68 (1H, dx2, J=4.9Hz and 4.9 Hz), 5.74 (2H, br s), 5.91 and 5.95 (1H, sx2), 6.61 and 6.65 (1H, dx2, J=9.3Hz and 9.3Hz), 6.67 and 6.83 (1H, dx2, J=7.8Hz and 7.8Hz), 6.87 and 6.92 (1H, sx2), 7.13 and 7.17 (1H, dx2, J=7.8Hz and 7.8Hz), 7.90-8.37 (5H, m), 8.88 (1H, m), 9.09 (1H, m) |
| 6 | 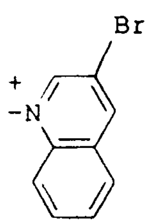 | M.p. 205-210°C (decomposed)<br>IR $\nu_{max}^{nujol}$ $cm^{-1}$: 3340, 3200, 1770, 1660, 1610<br>NMR ($D_2O$) δ: 2.62 and 2.69 (1H, dx2, J=17Hz and 17Hz), 3.22 and 3.28 (1H, dx2, J=17Hz and 17 Hz), 4.97 (1H, d, J=4.9Hz), 5.64 and 5.69 (1H, dx2, J=4.9Hz and 4.9 Hz), 5.77 (2H, br s), 5.91 and 5.96 (1H, sx2), 6.64 and 6.65 (1H, dx2, J=9.8Hz and 9.8Hz), 6.70 and 6.88 (1H, dx2, J=8.3Hz and 8.3Hz), 6.91 and 6.95 (1H, sx2), 7.21 (1H, d, J=8.3Hz), 8.15 (1H, m), 8.03-8.38 (4H, m), 9.15 (1H, s), 9.20 and 9.34 (1H, sx2) |

| | | |
|---|---|---|
| 7 | 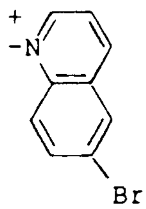 | M.p. 200-210°C (decomposed)<br>IR $\nu^{nujol}_{max}$ $cm^{-1}$: 3350, 3200, 1770, 1660 1610<br>NMR ($D_2O$) δ: 2.57 and 2.66 (1H, dx2, J=18Hz and 18Hz), 3.22 (1H, d, J=18Hz), 4.97 (1H, d, J=4.9Hz), 5.63 and 5.68 (1H, dx2, J=4.9Hz and 4.9Hz), 5.74 (2H, br s), 5.91 and 5.96 (1H, sx2), 6.61 and 6.65 (1H, dx2, J=10Hz and 10Hz), 6.65 and 6.83 (1H, dx2, J=7.3Hz and 8.3 Hz), 6.88 and 6.93 (1H, sx2), 7.13 and 7.16 (1H, dx2, J=7.3Hz and 8.3Hz), 7.96-8.31 (5H, m), 8.85 (1H, m), 9.13 (1H, m) |
| 8 | 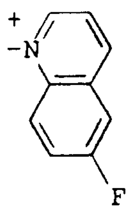 | M.p. 190-200°C (decomposed)<br>IR $\nu^{nujol}_{max}$ $cm^{-1}$: 3340, 3200, 1770, 1660, 1610<br>MS (m/z): 790 ($MNa^+$), 768 ($MH^+$)<br>NMR ($D_2O$) δ: 2.58 and 2.67 (1H, dx2, J=18Hz and 18Hz), 3.18 and 3.23 (1H, dx2, J=18Hz and 18Hz), 4.95 and 4.97 (1H, dx2, J=4.9Hz and 4.9Hz), 5.63 and 5.69 (1H, dx2, J=4.9Hz and 4.9Hz), 5.79 (2H, br s), 5.93 (1H, s), 6.63 and 6.66 (1H, dx2, J=9.8Hz and 9.8Hz), 6.71 and 6.87 (1H, dx2, J=8.8Hz and 8.8Hz), 6.90 and 6.93 (1H, sx2), 7.13 and 7.18 (1H, dx2, J=8.8Hz and 8.8Hz), 7.85-8.09 (3H, m), 8.20 and 8.29 (1H, dx2, J=9.8Hz and 9.8Hz), 8.43 (1H, m), 8.98 (1H, m), 9.10 (1H, d, J=5.4Hz) |
| 9 | 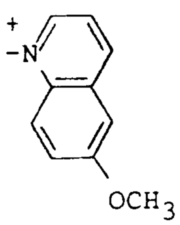 | M.p. 190-200°C (decomposed)<br>IR $\nu^{nujol}_{max}$ $cm^{-1}$: 3330, 3200, 1770, 1660, 1610<br>MS (m/z): 802 ($MNa^+$), 780 ($MH^+$)<br>NMR ($D_2O$) δ: 2.56 and 2.65 (1H, d, J=18Hz and 18Hz), 3.14 and 3.17 (1H, dx2, J=18Hz and 18Hz), 3.87 and 3.89 (3H, sx2), 4.95 and 4.96 (1H, dx2, J=4.4Hz and 4.4Hz), 5.63 and 5.68 (1H, dx2, J=4.4Hz and 4.4Hz), 5.69 (1H, br s), 5.89 and 5.92 (1H, sx2), 6.56 and 6.61 (1H, dx2, J=8.8Hz and 9.3 Hz), 6.64 and 6.79 (1H, dx2, J=8.3Hz and 8.3Hz), 6.84 and 6.90 (1H, sx2), 7.11 and 7.15 (1H, dx2, J=8.3Hz and 8.3Hz), 7.40 (1H, m), 7.63 (1H, m), 7.85 (1H, m), 8.13-8.29 (2H, m), 8.74 (1H, m), 8.87 (1H, m) |

| | | |
|---|---|---|
| 10 | 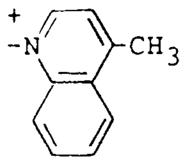 | M.p. 195-205°C (decomposed)<br>IR $\nu_{max}^{nujol}$ $cm^{-1}$: 3350, 3200, 1770, 1650 1600<br>MS (m/z): 786 ($MNa^+$), 764 ($MH^+$)<br>NMR ($D_2O$) δ: 2.58 and 2.67 (1H, dx2, J=18Hz and 18Hz), 2.89 and 2.90 (3H, sx2), 3.13 and 3.16 (1H, dx2, J=18Hz and 18Hz), 4.92 and 4.94 (1H, dx2, J=4.9Hz and 4.9Hz), 5.59 (1H, br d, J=14Hz), 5.61 and 5.66 (1H, dx2, J=4.9Hz and 4.9Hz), 5.76 (1H, br d, J=14Hz), 5.91 and 5.93 (1H, sx2), 6.57 and 6.62 (1H, dx2, J=9.8Hz and 9.8Hz), 6.65 and 6.83 (1H, dx2, J=8.3Hz and 8.3Hz), 6.88 and 6.92 (1H, sx2), 7.13 and 7.17 (1H, dx2, J=8.3Hz and 8.8Hz), 7.83 (2H, m), 8.00-8.29 (4H, m), 8.85 (1H, m) |
| 11 | 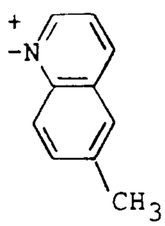 | M.p. 195-205°C (decomposed)<br>IR $\nu_{max}^{nujol}$ $cm^{-1}$: 3350, 3200, 1770, 1650, 1610<br>MS (m/z): 786 ($MNa^+$), 764 ($MH^+$)<br>NMR ($D_2O$) δ: 2.43 and 2.46 (3H, sx2), 2.57 and 2.66 (1H, dx2, J=18Hz and 18Hz), 3.13 and 3.15 (1H, dx2, J=18Hz and 18Hz), 4.95 and 4.97 (1H, dx2, J=4.4Hz and 4.4Hz), 5.63 and 5.69 (1H, dx2, J=4.4Hz and 4.4 Hz), 5.69 (2H, br s), 5.90 (1H, s), 6.55 and 6.61 (1H, dx2, J=9.8Hz and 9.8Hz), 6.70 and 6.83 (1H, dx2, J=8.3Hz and 8.3Hz), 6.85 and 6.90 (1H, sx2), 7.10 and 7.17 (1H, dx2, J=8.3Hz and 8.3Hz), 7.78-7.93 (3H, m), 8.13-8.29 (2H, m), 8.78 (1H, m), 9.00 (1H, m) |
| 11 | α-Isomer*1<br>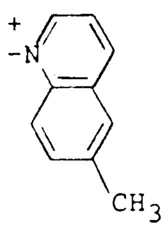 | M.p. >180°C (decomposed)<br>IR $\nu_{max}^{nujol}$ $cm^{-1}$: 3360, 3200, 1770, 1660, 1610<br>MS (m/z): 786 ($MNa^+$), 764 ($MH^+$)<br>NMR ($D_2O$) δ: 2.53 (3H, s), 2.63 (1H, d, J=18Hz), 3.15 (1H, d, J=18Hz), 4.95 (1H, d, J=4.4Hz), 5.64 (1H, d, J=4.4Hz), 5.67 (1H, d, J=16Hz), 5.82 (1H, d, J=16Hz), 5.91 (1H, s), 6.58 (1H, d, J=9.8Hz), 6.71 (1H, d, J=8.3Hz), 6.95 (1H, s), 7.10 (1H, d, J=8.3Hz), 7.90-7.98 (3H, m), 8.14-8.20 (2H, m), 8.88 (1H, d, J=8.3Hz), 9.02 (1H, d, J=5.9Hz) |

| | | |
|---|---|---|
| 11 | β-Isomer*1 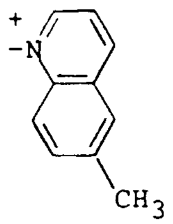 | M.p. >180°C (decomposed)<br>IR $\nu_{max}^{nujol}$ $cm^{-1}$: 3360, 3200, 1770, 1660, 1610<br>MS (m/z): 786 ($MNa^+$), 764 ($MH^+$)<br>NMR ($D_2O$) δ: 2.48 (3H, s), 2.54 (1H, d, J=18Hz), 3.13 (1H, d, J=18Hz), 4.94 (1H, d, J=4.4Hz), 5.68 (1H, d, J=16Hz), 5.69 (1H, d, J=4.4Hz), 5.77 (1H, d, J=16Hz), 5.93 (1H, s), 6.64 (1H, d, J=9.8Hz), 6.86 (1H, d, J=8.3Hz), 6.88 (1H, s), 7.18 (1H, d, J=8.3Hz), 7.85-7.93 (3H, m), 8.20 (1H, d, J=9.3Hz), 8.29 (1H, d, J=9.8Hz), 8.82 (1H, d, J=7.3Hz), 8.99 (1H, d, J=6.8Hz) |
| 12 | 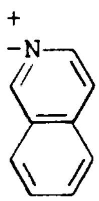 | M.p. 185-195°C (decomposed)<br>IR $\nu_{max}^{nujol}$ $cm^{-1}$: 3340, 3200, 1770, 1650, 1610<br>MS (m/z): 750 ($MH^+$)<br>NMR ($D_2O$) δ: 2.71 and 2.74 (1H, dx2, J=18Hz and 18Hz), 3.41 (1H, d, J=18Hz), 5.07 and 5.08 (1H, dx2, J=4.4Hz and 4.4Hz), 5.26 and 5.31 (1H, dx2, J=14Hz and 14Hz), 5.61 (1H, d, J=14Hz), 5.65 and 5.72 (1H, dx2, J=4.4Hz and 4.4Hz), 5.86 and 5.89 (1H, sx2), 6.50 and 6.73 (1H, dx2, J=7.8Hz and 7.8Hz), 6.54 and 6.57 (1H, dx2, J=9.8Hz and 9.8 Hz), 6.85 and 6.89 (1H, sx2), 6.99 and 7.10 (1H, dx2, J=7.8Hz and 7.8Hz), 7.68-8.24 (6H, m), 8.49 (1H, m), 9.59 (1H, br s) |
| 13 | 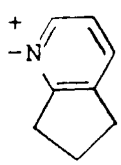 | M.p. >175°C (decomposed)<br>IR $\nu_{max}^{nujol}$ $cm^{-1}$: 3350, 3200, 1770, 1660, 1610<br>MS (m/z): 740 ($MH^+$)<br>NMR ($D_2O$) δ: 2.29 (2H, m), 2.64 and 2.69 (1H, dx2, J=18Hz and 18Hz), 3.11-3.30 (5H, m), 4.96 and 4.98 (1H, dx2, J=4.9Hz and 4.9Hz), 5.21 (1H, d, J=15Hz), 5.36 and 5.38 (1H, dx2, J=15Hz and 15Hz), 5.64 and 5.71 (1H, dx2, J=4.9Hz and 4.9Hz), 5.97 (1H, s), 6.69 and 6.72 (1H, dx2, J=9.8Hz and 9.8Hz), 6.91 and 7.01 (1H, dx2, J=7.8Hz and 8.3Hz), 6.97 and 6.98 (1H, sx2), 7.19 and 7.23 (1H, dx2, J=7.8Hz and 8.3Hz), 7.68-7.79 (1H, m), 8.23-8.45 (3H, m) |

| | | |
|---|---|---|
| 14 | 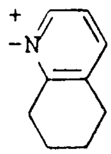 | M.p. >175°C (decomposed)<br>IR $\nu_{max}^{nujol}$ $cm^{-1}$: 3350, 3200, 1770, 1660, 1610<br>MS (m/z): 754 ($MH^+$)<br>NMR ($D_2O$) δ: 1.79-1.91 (4H, m), 2.63 and 2.67 (1H, dx2, J=18Hz and 18Hz), 2.93 (4H, m), 3.15 and 3.17 (1H, dx2, J=18Hz and 18Hz), 4.97 and 4.98 (1H, dx2, J=4.9Hz and 4.9Hz), 5.23 (2H, br s), 5.64 and 5.71 (1H, dx2, J=4.9Hz and 4.9Hz), 5.97 (1H, s), 6.69 and 6.73 (1H, dx2, J=9.8Hz and 9.8Hz), 6.89 and 7.00 (1H, dx2, J=7.3Hz and 7.8Hz), 6.96 and 6.97 (1H, sx2), 7.20 and 7.24 (1H, dx2, J=7.3Hz and 7.8Hz), 7.60-7.78 (1H, m), 8.14 (1H, m), 8.26 and 8.35 (1H, dx2, J=9.8Hz and 9.8Hz), 8.47 (1H, d, J=6.3Hz) |
| 15 | 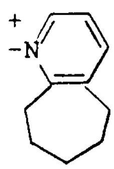 | M.p. >160°C (decomposed)<br>IR $\nu_{max}^{nujol}$ $cm^{-1}$: 3350, 3200, 1770, 1660, 1610<br>MS (m/z): 790 ($MNa^+$), 768 ($MH^+$)<br>NMR ($D_2O$) δ: 1.50-1.90 (6H, m), 2.58 and 2.63 (1H, dx2, J=18Hz and 18Hz), 2.90-3.30 (5H, m), 4.93 (1H, m), 5.34, 5.73 (2H, ABq, J=17Hz), 5.61 and 5.67 (1H, dx2, J=4.4Hz and 4.4Hz), 5.97 (1H, s), 6.71 and 6.74 (1H, d, J=9.8Hz and 9.8Hz), 6.94 and 7.02 (1H, dx2, J=8.3Hz and 8.3Hz), 6.99 (1H, s), 7.20 and 7.24 (1H, dx2, J=8.3Hz and 8.3Hz), 7.70 (1H, m), 8.21 (1H, d, J=9.3Hz), 8.26 ad 8.35 (1H, dx2, J=9.8Hz and 9.8Hz), 8.50 (1H, d, J=5.4Hz) |
| 16 | 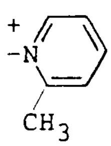 | M.p. >160°C (decomposed)<br>IR $\nu_{max}^{nujol}$ $cm^{-1}$: 3350, 3200, 1770, 1660, 1610<br>MS (m/z): 736 ($MNa^+$), 714 ($MH^+$)<br>NMR ($D_2O$) δ: 2.61 and 2.66 (1H, dx2, J=18Hz and 18Hz), 2.79 and 2.80 (3H, sx2), 3.21 and 3.24 (1H, dx2, J=18Hz and 18Hz), 4.96 and 4.99 (1H, dx2, J=4.9Hz and 4.9Hz), 5.27 (1H, d, J=15Hz), 5.45 (1H, d, J=15Hz), 5.63 and 5.71 (1H, dx2, J=4.9Hz and 4.9Hz), 5.98 (1H, s), 6.70 and 6.73 (1H, dx2, J=9.8Hz and 9.8Hz), 6.90 and 7.01 (1H, dx2, J=8.3Hz and 8.3Hz), 7.00 (1H, s), 7.18 and 7.23 (1H, dx2, J=8.3Hz and 8.3Hz), 7.91 (2H, m), 8.22-8.46 (2H, m), 8.67 (1H, d, J=5.9Hz) |

| | | |
|---|---|---|
| 17 | 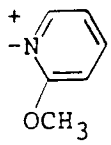 | M.p. >175°C (decomposed)<br>IR $\nu_{max}^{nujol}$ $cm^{-1}$: 3360, 3200, 1770, 1660, 1640, 1610<br>MS (m/z): 752 ($MNa^+$), 730 ($MH^+$)<br>NMR ($D_2O$) δ: 2.76 and 2.81 (1H, dx2, J=18Hz and 18Hz), 3.17 and 3.20 (1H, dx2, J=18Hz and 18Hz), 4.24 (3H, s), 4.87 (1H, s, J=4.9Hz), 4.90 (1H, d, J=15Hz), 5.60 and 5.67 (1H, dx2, J=4.9Hz and 4.9Hz), 5.66 (1H, d, J=15Hz), 5.98 (1H, s), 6.71 and 6.73 (1H, dx2, J=9.8Hz and 9.8Hz), 6.99 (1H, s), 7.00 and 7.04 (1H, dx2, J=8.3Hz and 8.3Hz), 7.21 ad 7.24 (1H, dx2, J=8.3Hz and 8.3Hz), 7.46-7.57 (2H, m), 8.26 and 8.34 (1H, dx2, J=9.8Hz and 9.8Hz), 8.34-8.46 (2H, m) |
| 18 | 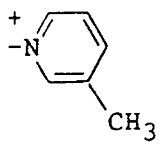 | M.p. >175°C (decomposed)<br>IR $\nu_{max}^{nujol}$ $cm^{-1}$: 3340, 3200, 1770, 1660, 1610<br>MS (m/z): 736 ($MNa^+$), 714 ($MH^+$)<br>NMR ($D_2O$) δ: 2.52 and 2.55 (3H, sx2), 2.59 and 2.65 (1H, dx2, J=18Hz and 18Hz), 3.35 and 3.36 (1H, dx2, J=18Hz and 18Hz), 4.99 and 5.00 (1H, dx2, J=4.9Hz and 4.9Hz), 5.21 and 5.24 (1H, dx2, J=15Hz and 15Hz), 5.44 (1H, d, J=15Hz), 5.63 and 5.71 (1H, dx2, J=4.9Hz and 4.9Hz), 5.96 (1H, s), 6.69 (1H, d, J=9.8Hz), 6.83 and 7.13 (1H, dx2, J=8.3Hz and 8.3Hz), 6.99 (1H, s), 7.22 (1H, d, J=8.3Hz), 7.95 (1H, m), 8.23 and 8.32 (1H, dx2, J=9.8Hz and 9.8Hz), 8.31-8.40 (1H, m), 8.70 (1H, d, J=7.8Hz), 8.72 (1H, br s) |
| 19 | 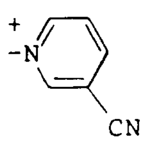 | M.p. >180°C (decomposed)<br>IR $\nu_{max}^{nujol}$ $cm^{-1}$: 3340, 3200, 1770, 1660, 1610<br>MS (m/z): 621 ($MH^+$-3-cyanopyridine)<br>NMR ($D_2O$) δ: 2.69 and 2.76 (1H, dx2, J=18Hz and 18Hz), 3.45 and 3.47 (1H, dx2, J=18Hz and 18Hz), 5.02 and 5.03 (1H, dx2, J=4.9Hz and 4.9Hz), 5.33 and 5.34 (1H, dx2, J=14Hz and 14Hz), 5.66 (1H, d, J=14Hz), 5.66 and 5.74 (1H, dx2, J=4.9Hz and 4.9Hz), 5.96 (1H, s), 6.69 (1H, d, J=9.3Hz), 6.88 and 7.00 (1H, dx2, J=8.3Hz and 8.3Hz), 6.99 (1H, s), 7.16 and 7.22 (1H, dx2, J=8.3Hz and 8.3Hz), 8.21-8.38 (2H, m), 8.99 (1H, br d), 9.25 (1H, d, J=4.9Hz), 9.56 (1H, br d) |

| | | |
|---|---|---|
| 20 | 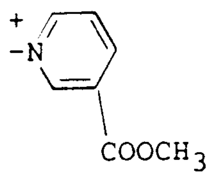 | M.p. >175°C (decomposed)<br>IR $\nu_{max}^{nujol}$ $cm^{-1}$: 3330, 3200, 1770, 1740 1650, 1610<br>MS (m/z): 780 ($MNa^+$), 758 ($MH^+$)<br>NMR ($D_2O$) δ: 2.66 and 2.75 (1H, dx2, J=18Hz and 18Hz), 3.46 and 3.47 (1H, dx2, J=18Hz and 18Hz), 4.01 and 4.04 (3H, sx2), 5.03 and 5.04 (1H, dx2, J=4.9Hz and 4.9Hz), 5.28 and 5.33 (1H, dx2, J=15Hz and 15Hz), 5.61 (1H, d, J=15Hz), 5.65 and 5.72 (1H, dx2, J=4.9Hz and 4.9Hz), 5.94 (1H, s), 6.66 and 6.68 (1H, dx2, J=9.8Hz and 9.8Hz), 6.83 and 6.97 (1H, dx2, J=8.3Hz and 8.3Hz), 6.93 and 6.95 (1H, sx2), 7.14 and 7.20 (1H, dx2, J=8.3Hz and 8.3Hz), 8.17-8.23 (1H, m), 8.24 and 8.31 (1H, dx2, J=9.8Hz and 9.8Hz), 8.98-9.05 (1H, m), 9.14 (1H, d, J=6.4Hz), 9.55 (1H, br s) |
| 21 | 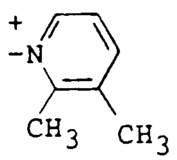 | M.p. >160°C (decomposed)<br>IR $\nu_{max}^{nujol}$ $cm^{-1}$: 3340, 3200, 1770, 1660, 1610<br>MS (m/z): 750 ($MNa^+$), 728 ($MH^+$)<br>NMR ($D_2O$) δ: 2.50 (3H, s), 2.68 (4H, m), 3.13 and 3.15 (1H, dx2, J=18Hz and 18Hz), 4.95 and 4.98 (1H, dx2, J=4.9Hz and 4.9Hz), 5.27-5.56 (2H, m), 5.63 and 5.70 (1H, dx2, J=4.9Hz and 4.9Hz), 5.98 (1H, s), 6.70 and 6.73 (1H, dx2, J=9.8Hz and 9.8Hz), 6.92 and 7.01 (1H, dx2, J=8.3Hz and 8.3Hz), 6.99 (1H, s), 7.20 and 7.23 (1H, dx2, J=8.3Hz and 8.3Hz), 7.77 (1H, m), 8.24 (1H, m), 8.26 and 8.31 (1H, dx2, J=9.8Hz and 9.8Hz), 8.50 (1H, d, J=5.7Hz) |

| | | |
|---|---|---|
| 22 | 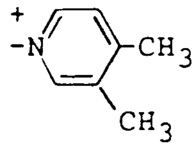 | M.p. >160°C (decomposed)<br>IR $\nu_{max}^{nujol}$ $cm^{-1}$: 3350, 3200, 1770, 1660, 1610<br>MS (m/z): 750 ($MNa^+$), 728 ($MH^+$)<br>NMR ($D_2O$) $\delta$: 2.35 and 2.41 (3H, sx2), 2.48 and 2.53 (3H, sx2), 2.63 and 2.65 (1H, dx2, J=18Hz and 18Hz), 3.34 (1H, d, J=18Hz), 5.00 (1H, d, J=4.9Hz), 5.10 and 5.14 (1H, dx2, J=14Hz and 14Hz), 5.35 (1H, d, J=14Hz), 5.62 and 5.69 (1H, dx2, J=4.9Hz and 4.9Hz), 5.94 and 5.95 (1H, sx2), 6.68 (1H, d, J=10Hz), 6.73 and 6.95 (1H, dx2, J=8.3Hz and 8.3Hz), 6.92 and 6.97 (1H, sx2), 7.11 and 7.21 (1H, dx2, J=8.3Hz and 8.3Hz), 7.73 and 7.79 (1H, dx2, J=6.4Hz and 6.4Hz), 8.24 and 8.32 (1H, dx2, J=10Hz and 10Hz), 8.51 (1H, d, J=6.4Hz), 8.53 (1H, s) |
| 23 | 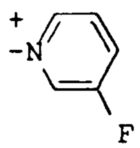 | M.p. 185-190°C (decomposed)<br>IR $\nu_{max}^{nujol}$ $cm^{-1}$: 3350, 3200, 1770, 1660, 1610<br>MS (m/z): 740 ($MNa^+$), 718 ($MH^+$)<br>NMR ($D_2O$) $\delta$: 2.65 and 2.71 (1H, dx2, J=18Hz and 18Hz), 3.40 and 3.42 (1H, dx2, J=18Hz and 18Hz), 5.00 and 5.01 (1H, dx2, J=4.9Hz and 4.9Hz), 5.29 and 5.32 (1H, dx2, J=15Hz and 15Hz), 5.59 (1H, d, J=15Hz), 5.64 and 5.73 (1H, dx2, J=4.9Hz and 4.9Hz), 5.96 (1H, s), 6.69 (1H, d, J=9.8Hz), 6.85 and 7.00 (1H, dx2, J=8.3Hz and 8.3Hz), 6.99 (1H, s), 7.14 and 7.22 (1H, dx2, J=8.3Hz and 8.3Hz), 8.18 (1H, m), 8.24 and 8.32 (1H, dx2, J=9.8Hz and 9.8Hz), 8.50 (1H, m), 8.87 (1H, d, J=5.4Hz), 9.11 (1H, br s) |

| | | |
|---|---|---|
| 24 | 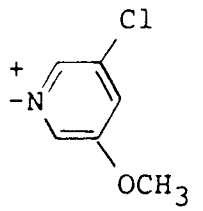 | M.p. >180°C (decomposed)<br>IR $\nu_{max}^{nujol}$ $cm^{-1}$: 3350, 3200, 1770, 1660, 1610<br>MS (m/z): 644 ($MNa^+$-3-chloro-5-methoxypyridine)<br>621 ($MH^+$-3-chloro-5-methoxypyridine)<br>NMR ($D_2O$) δ: 2.67 and 2.73 (1H, dx2, J=18Hz and 18Hz), 3.43 (1H, d, J=18Hz), 3.95 and 4.01 (3H, sx2), 5.04 (1H, d, J=4.9Hz), 5.14 and 5.19 (1H, dx2, J=15Hz and 15Hz), 5.50 (1H, d, J=15Hz), 5.65 and 5.71 (1H, dx2, J=4.9Hz and 4.9Hz), 5.95 (1H, s), 6.69 and 6.70 (1H, dx2, J=9.8Hz and 9.8Hz), 6.85 and 6.98 (1H, dx2, J=8.3Hz and 8.3Hz), 6.94 and 6.96 (1H, sx2), 7.17 and 7.22 (1H, dx2, J=8.3Hz and 8.3Hz), 8.15 (1H, m), 8.25 and 8.33 (1H, dx2, J=9.8Hz and 9.8Hz), 8.69 (2H, m) |
| 25 | 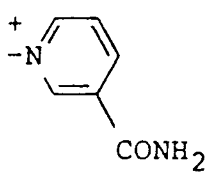 | M.p. >190°C (decomposed)<br>IR $\nu_{max}^{nujol}$ $cm^{-1}$: 3330, 3200, 1770, 1660, 1610<br>MS (m/z): 765 ($MNa^+$), 743 ($MH^+$)<br>NMR ($D_2O$) δ: 2.63 and 2.71 (1H, dx2, J=18Hz and 18Hz), 3.42 (1H, br d, J=18Hz), 5.01 (1H, m), 5.29 and 5.32 (1H, dx2, J=14Hz and 14Hz), 5.61 (1H, d, J=14Hz), 5.63 and 5.72 (1H, dx2, J=4.9Hz and 4.9Hz), 5.95 (1H, s), 6.66 and 6.68 (1H, dx2, J=9.8Hz and 9.8Hz), 6.77 and 6.96 (1H, dx2, J=8.3Hz and 8.3Hz), 6.97 (1H, s), 7.11 and 7.20 (1H, dx2, J=8.3Hz and 8.3Hz), 8.19-8.32 (2H, m), 8.91 (1H, m), 9.11 (1H, d, J=5.9Hz), 9.37 (1H, s) |

| | | |
|---|---|---|
| 26 | 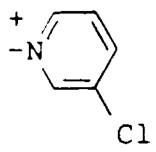 | M.p. >175°C (decomposed)<br>IR $\nu_{max}^{nujol}$ $cm^{-1}$: 3350, 3200, 1770, 1660, 1610<br>MS (m/z): 758 ($MNa^{+}$+2), 756 ($MNa^{+}$), 736 ($MH^{+}$+2), 734 ($NH^{+}$)<br>NMR ($D_2O$) δ: 2.66 and 2.71 (1H, dx2, J=18Hz and 18Hz), 3.41 and 3.42 (1H, dx2, J=18Hz and 18Hz), 5.01 and 5.02 (1H, dx2, J=4.9Hz and 4.9Hz), 5.24 and 5.26 (1H, dx2, J=15Hz and 15Hz), 5.55 (1H, d, J=15Hz), 5.65 and 5.73 (1H, dx2, J=4.9Hz and 4.9Hz), 5.96 (1H, s), 6.69 (1H, d, J=9.8Hz), 6.85 and 7.00 (1H, dx2, J=8.3Hz and 8.3Hz), 6.97 (1H, s), 7.15 and 7.22 (1H, dx2, J=8.3Hz and 8.3Hz), 8.03-8.13 (1H, m), 8.23 and 8.32 (1H, dx2, J=9.8Hz and 9.8Hz), 8.59-8.66 (1H, m), 8.91 (1H, d, J=6.4Hz), 9.14 (1H, br s) |
| 26 | α-Isomer*2<br>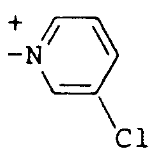 | M.p. >170°C (decomposed)<br>IR $\nu_{max}^{nujol}$ $cm^{-1}$: 3350, 3200, 1770, 1660, 1610<br>MS (m/z): 758 ($MNa^{+}$+2), 756 ($MNa^{+}$), 736 ($MH^{+}$+2), 734 ($MH^{+}$)<br>NMR ($D_2O$) δ: 2.69, 3.42 (2H, ABq, J=18Hz), 5.01 (1H, d, J=4.9Hz), 5.27, 5.56 (2H, ABq, J=14Hz), 5.64 (1H, d, J=4.9Hz), 5.96 (1H, s), 6.69 (1H, d, J=9.8Hz), 6.85 (1H, d, J=7.8Hz), 7.01 (1H, s), 7.15 (1H,d, J=7.8Hz), 8.12 (1H, dd, J=8.3Hz, 5.9Hz), 8.22 (1H, d, J=9.8Hz), 8.67 (1H, d, J=8.3Hz), 8.92 (1H, d, J=5.9Hz), 9.14 (1H, s) |

| | | |
|---|---|---|
| 26 | β-Isomer*2 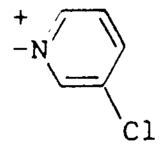 | M.p. >170°C (decomposed)<br>IR $\nu_{max}^{nujol}$ $cm^{-1}$: 3350, 3200, 1770, 1660, 1610<br>MS (m/z): 736 ($MNa^+$), 734 ($MH^+$)<br>NMR ($D_2O$) δ: 2.63, 3.41 (2H, ABq, J=18Hz), 5.00 (1H, d, J=4.9Hz), 5.26, 5.56 (2H, ABq, J=14Hz), 5.72 (1H, d, J=4.9Hz), 5.97 (1H, s), 6.71 (1H, d, J=9.8Hz), 7.00 (1H, s), 7.02 (1H, d, J=7.8Hz), 7.23 (1H, d, J=7.8Hz), 8.10 (1H, dd, J=8.3Hz, 5.9Hz), 8.33 (1H, d, J=9.8Hz), 8.63 (1H, d, J=8.3Hz), 8.91 (1H, d, J=5.9Hz), 9.15 (1H, s) |

*1: Each isomer is isolated by high performance liquid chromatography [solid phase; $C_{18}$-silica gel (YMC-Pack S-343), eluent; acetonitril/30 mM phosphate buffer (pH 7.0) = 1 : 9)].

*2: Each isomer is isolated by high performance liquid chromatography [solid phase; $C_{18}$-silica gel (YMC-Pack D-ODS-10), eluent; acetonitril/30 mM phosphate buffer (pH 7.0) = 6 : 94].

Example 27

(1) To dimethylformamide (0.38 g) are added dropwise phosphorus oxychloride (0.48 g) and methylene chloride (2.2 ml) with ice-cooling under argon atmosphere, and the mixture is stirred at room temperature for 1.5 hour, and cooled to -55 to -50 °C. To the mixture is added dropwise a solution of 2-(2-tritylaminothiazol-4-yl)-2-[(8-diphenylmethyloxy-2-oxo-1H-quinolin-5-yl)(diphenylmethyloxycarbonyl)-methyloxyimino]acetic acid (2.2 g) in methylene chloride (54 ml). The mixture is stirred at -65 to -55 °C for 1.5 hour, and then at -55 to -50 °C for 30 minutes (the resulting solution is referred to as "Reaction solution A"). Separately, bis(trimethylsilyl)acetamide (3.08 g) is added to a suspension of 7β-amino-3-(3-amino-2-methyl-1-pyridiniomethyl)-3-cephem-4-carboxylate (1.57 g) in methylene chloride (32 ml) with ice-cooling under argon atmosphere, and the mixture is stirred at the same temperature for two hours (the resulting solution is referred to as "Reaction solution B"). The Reaction solution B is added dropwise to the Reaction solution A, and the mixture is stirred at -55 to -30 °C for 40 minutes, and then at -30 °C for 1.5 hour. After completion of the reaction, the mixture is poured into water, and evaporated to remove methylene chloride. The resulting precipitates are collected by filtration, washed with water, and dried. The resulting residue is added to a mixture of anisole (10 ml) and trifluoroacetic acid (10 ml) at 15 to 20 °C, and the mixture is stirred at room temperature for two hours. To the mixture is added isopropyl ether (180 ml) under ice-cooling, and the mixture is stirred for 15 minutes. The precipitates are collected by filtration, washed with isopropyl ether, dried, and suspended in water. The pH value of this suspension is adjusted to pH 7.7 with saturated aqueous sodium hydrogen carbonate solution, and purified by HP-20 column chromatography (eluent; water and 20 % aqueous methanol). The fractions containing the desired compound are combined, concentrated, and lyophilized to give 7β-{(Z)-2-(2-aminothiazol-4-yl)-2-[(8-hydroxy-2-oxo-1H-quinolin-5-yl)(carboxy)methyloxyimino]acetamido}-3-(3 amino-2-methyl-1-pyridiniomethyl)-3-cephem-4-carboxylic acid sodium salt (0.75 g) as light yellow powder.

M.p. >160 ° C (decomposed)

IR (Nujol): 3360, 3200, 1770, 1650, 1610 $cm^{-1}$

MS (m/z): 751 ($MNa^+$), 729 ($MH^+$)

NMR ($D_2O$) $\delta$: 2.50 and 2.52 (3H, sx2), 2.54 and 2.57 (1H, dx2, J = 17Hz and 18Hz), 3.06 and 3.09 (1H, dx2, J = 17Hz and 18Hz), 4.92 and 4.95 (1H, dx2, J = 4.4Hz and 4.4Hz), 5.22 (1H, d, J = 15Hz), 5.50 and 5.51 (1H, d, J = 15Hz and 15Hz), 5.60 and 5.68 (1H, dx2, J = 4.4Hz and 4.4Hz), 5.99 (1H, s), 6.71 and 6.74 (1H, dx2, J = 9.8Hz and 9.8Hz), 6.91 and 7.01 (1H, dx2, J = 8.3Hz and 8.3Hz), 7.01 (1H, s), 7.18 and 7.22 (1H, dx2, J = 8.3Hz and 8.3Hz), 7.52-7.62 (1H, m), 7.69 and 7.71 (1H, dx2, J = 8.3Hz and 8.3Hz), 8.00 (1H, d, J = 6.3Hz), 8.24 and 8.34 (1H, d, J = 9.8Hz and 9.8Hz)

(2) The product (70 mg) obtained above is subjected to high performance liquid chromatography to isolate each isomer, and purified by HP-20 column chromatography to give a-isomer and $\beta$-isomer, separately.

Rt of $\alpha$-isomer: 45 minutes

Rt of $\beta$-isomer: 75 minutes

[Conditions]

Solid phase; $C_{18}$-silica gel (tradename: YMC-Pack D-ODS-10)
Eluent; Acetonitril : 30 mM phosphate buffer (pH 7.0) = 6 : 94
Flow rate; 5 ml/minute

$\alpha$-Isomer

Yield: 20 mg

M.p. >160 ° C (decomposed)

IR (Nujol): 3360, 3200, 1770, 1650, 1610 $cm^{-1}$

MS (m/z): 751 ($MNa^+$), 729 ($MH^+$)

NMR ($D_2O$) $\delta$: 2.52 (3H, s), 2.57, 3.09 (2H, ABq, J = 18Hz), 4.95 (1H, d, J = 4.4Hz), 5.22, 5.51 (2H, ABq, J = 15Hz), 5.60 (1H, d, J = 4.4Hz), 5.98 (1H, s), 6.71 (1H, d, J = 9.8Hz), 6.91 (1H, d, J = 8.3Hz), 7.01 (1H, s), 7.18 (1H, d, J = 8.3Hz), 7.59 (1H, dd, J = 8 3Hz, 5.9Hz), 7.71 (1H, d, J = 8.3Hz), 8.00 (1H, d, J = 5.9Hz), 8.24 (1H, d, J = 9.8Hz)

$\beta$-Isomer

Yield: 20 mg

M.p. >160 ° C (decomposed)

IR (Nujol): 3360, 3200, 1770, 1660, 1610 $cm^{-1}$

MS (m/z): 751 ($MNa^+$), 729 ($MH^+$)

NMR ($D_2O$) $\delta$: 2.50 (3H, s), 2.54, 3.06 (2H, ABq, J = 17Hz), 4.92 (1H, d, J = 4.4Hz), 5.22, 5.50 (2H, ABq, J = 15Hz), 5.68 (1H, d, J = 4.4Hz), 5.99 (1H, s), 6.74 (1H, d, J = 9.8Hz), 7.01 (1H, s), 7.01 (1H, d, J = 8.3Hz), 7.22 (1H, d, J = 8.3Hz), 7.56 (1H, dd, J = 7.8Hz, 6.3Hz), 7.69 (1H, d, J = 7.8Hz), 8.00 (1H, d, J = 6.3Hz), 8.34 (1H, d, J = 9.8Hz)

Examples 28 - 33

2-(2-Tritylaminothiazol-4-yl)-2-[(8-diphenylmethyloxy-2-oxo-1H-quinolin-5-yl)-(diphenylmethyloxycarbonyl)methyloxyimino]acetic acid and the corresponding starting compounds are treated in the same manner as in Example 27 except that the removal of the protecting groups are carried out by using 80 % aqueous formic acid solution to give the compounds of the following Table 2.

Table 2

In the following Table 2, $R^5$ is $COO^-$ in Examples 28 - 31, and COONa in Examples 32 - 33.

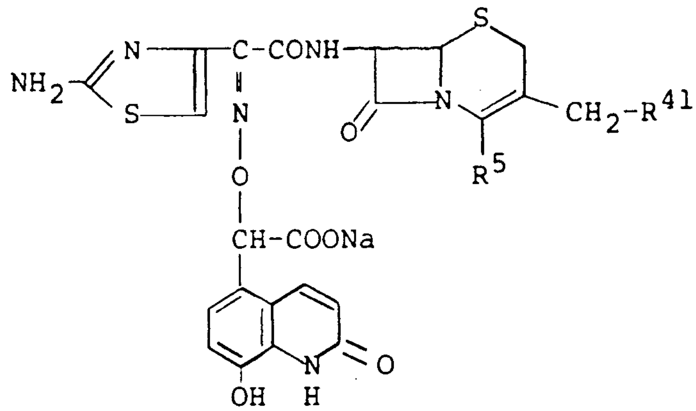

| Ex. No. | Compound | |
|---|---|---|
| | $R^{41}$ | Physicochemical properties |
| 28 | 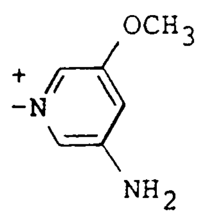 | M.p. >170°C (decomposed)<br>IR $\nu_{max}^{nujol}$ $cm^{-1}$: 3340, 3200, 1770, 1650, 1600<br>MS (m/z): 767 ($MNa^+$), 745 ($MH^+$)<br>NMR ($D_2O$) δ: 2.58 and 2.62 (1H, dx2, J=18Hz and 18Hz), 3.32 (1H, d, J=18Hz), 3.83 and 3.88 (3H, sx2), 4.70-4.90 (1H), 5.00 (1H, d, J=4.9Hz), 5.26 (1H, d, J=15Hz), 5.61 and 5.68 (1H, dx2, J=4.9Hz and 4.9Hz), 5.95 and 5.96 (1H, sx2), 6.68 and 6.69 (1H, dx2, J=9.8Hz and 9.8Hz), 6.80 and 6.96 (1H, dx2, J=7.8Hz and 7.8Hz), 6.96 and 6.97 (1H, sx2), 7.10 and 7.15 (1H, br sx2), 7.14 and 7.21 (1H, dx2, J=7.8Hz and 7.8Hz), 7.78 (1H, br s), 7.82 (1H, br s), 8.23 and 8.31 (1H, dx2, J=9.8Hz and 9.8Hz) |

| | | |
|---|---|---|
| 29 | 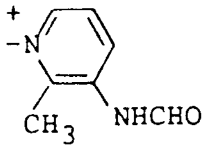 | M.p. >150°C (decomposed)<br>IR $\nu_{max}^{nujol}$ $cm^{-1}$: 3380, 3200, 1770, 1660, 1610<br>MS (m/z): 779 ($MNa^+$), 757 ($MH^+$)<br>NMR ($D_2O$) δ: 2.54 and 2.66 (1H, dx2, J=18Hz and 18Hz), 3.22 and 3.26 (1H, dx2, J=18Hz and 18Hz), 2.73 (3H, s), 4.96 and 5.00 (1H, dx2, J=4.9Hz and 4.9Hz), 5.37 and 5.39 (1H, dx2, J=16Hz and 16Hz), 5.54 (1H, d, J=16Hz), 5.64 and 5.70 (1H, dx2, J=4.9Hz and 4.9Hz), 5.99 (1H, s), 6.72 and 6.74 (1H, dx2, J=9.8Hz and 9.8Hz), 6.93 and 7.02 (1H, dx2, J=8.3Hz and 8.3Hz), 7.02 (1H, s), 7.19 and 7.22 (1H, dx2, J=8.3Hz and 8.3Hz), 7.90-8.02 (1H, m), 8.25 and 8.34 (1H, dx2, J=9.8Hz and 9.8Hz), 8.50 (1H, s), 8.63 and 8.67 (1H, dx2, J=5.9Hz and 6.4Hz), 8.73 (1H, d, J=8.3Hz) |
| 29 | α-Isomer*<br>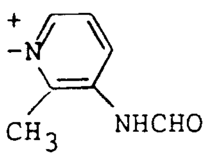 | M.p. >150°C (decomposed)<br>IR $\nu_{max}^{nujol}$ $cm^{-1}$: 3370, 3200, 1770, 1660, 1610<br>MS (m/z): 779 ($MNa^+$), 757 ($MH^+$)<br>NMR ($D_2O$) δ: 2.66, 3.25 (2H, ABq, J=18Hz), 2.73 (3H, s), 5.00 (1H, d, J=4.9Hz), 5.37, 5.54 (2H, ABq, J=16Hz), 5.64 (1H, d, J=4.9Hz), 5.98 (1H, s), 6.72 (1H, d, J=9.8Hz), 6.93 (1H, d, J=8.3Hz), 7.02 (1H, s), 7.19 (1H, d, J=8.3Hz), 7.99 (1H, dd, J=8.3Hz, 5.9Hz), 8.25 (1H, d, J=9.8Hz), 8.49 (1H, s), 8.63 (1H, d, J=5.9Hz), 8.73 (1H, d, J=8.3Hz) |
| 29 | β-Isomer*<br>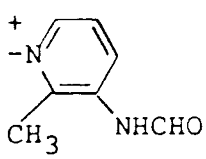 | M.p. >150°C (decomposed)<br>IR $\nu_{max}^{nujol}$ $cm^{-1}$: 3380, 3200, 1770, 1660, 1610<br>MS (m/z): 779 ($MNa^+$), 757 ($MH^+$)<br>NMR ($D_2O$) δ: 2.54, 3.22 (2H, ABq, J=18Hz), 2.73 (3H, s), 4.96 (1H, d, J=4.9Hz), 5.39, 5.53 (2H, ABq, J=16Hz), 5.70 (1H, d, J=4.9Hz), 5.99 (1H, s), 6.74 (1H, d, J=9.8Hz), 7.02 (1H, s), 7.02 (1H, d, J=8.3Hz), 7.22 (1H, d, J=8.3Hz), 7.95 (1H, dd, J=8.8Hz, 6.4Hz), 8.34 (1H, d, J=9.8Hz), 8.50 (1H, s), 8.67 (1H, d, J=6.4Hz), 8.72 (1H, d, J=8.8Hz) |

| | | |
|---|---|---|
| 30 | 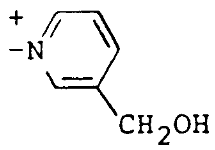 | M.p. >165°C (decomposed)<br>IR $\nu_{max}^{nujol}$ $cm^{-1}$: 3300, 3200, 1770, 1660, 1610<br>MS (m/z): 752 ($MNa^+$), 730 ($MH^+$)<br>NMR ($D_2O$) δ: 2.61 and 2.67 (1H, dx2, J=18Hz and 18Hz), 3.36 and 3.38 (1H, dx2, J=18Hz and 18Hz), 4.88 and 4.90 (2H, sx2), 4.99 and 5.00 (1H, dx2, J=4.9Hz and 4.9Hz), 5.25 and 5.27 (1H, dx2, J=14Hz and 14Hz), 5.52 (1H, d, J=14Hz), 5.63 and 5.71 (1H, dx2, J=4.9Hz and 4.9Hz), 5.96 (1H, s), 6.69 (1H, d, J=9.8Hz), 6.82 and 6.99 (1H, dx2, J=8.3Hz and 8.3Hz), 6.99 (1H, s), 7.12 and 7.21 (1H, dx2, J=8.3Hz and 8.3Hz), 8.07 (1H, m), 8.22 and 8.31 (1H, dx2, J=9.8Hz and 9.8Hz), 8.51 (1H, m), 8.83 (1H, d, J=5.9Hz), 8.92 (1H, s) |
| 31 | 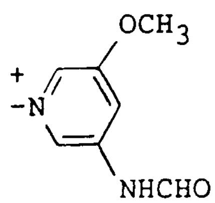 | M.p. >170°C (decomposed)<br>IR $\nu_{max}^{nujol}$ $cm^{-1}$: 3350, 3200, 1770, 1660, 1610<br>MS (m/z): 795 ($MNa^+$), 773 ($MH^+$)<br>NMR ($D_2O$) δ: 2.61 and 2.71 (1H, dx2, J=18Hz and 18Hz), 3.40 and 3.44 (1H, dx2, J=18Hz and 18Hz), 3.92 and 3.95 (3H, sx2), 5.03-5.07 (2H, m), 5.49 (1H, d, J=14Hz), 5.62 and 5.71 (1H, dx2, J=4.9Hz and 4.4Hz), 5.94 (1H, s), 6.64 and 6.69 (1H, dx2, J=9.8Hz and 9.8Hz), 6.77 and 6.93 (1H, dx2, J=8.3Hz and 8.3Hz), 6.91 and 6.93 (1H, sx2), 7.13 and 7.21 (1H, dx2, J=8.3Hz and 8.3Hz), 7.94 and 8.01 (1H, br sx2), 8.22-8.45 (3H, m), 8.97 and 9.00 (1H, br sx2) |

| | | |
|---|---|---|
| 32 | 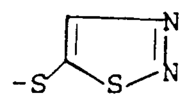 | M.p. 175-185°C (decomposed)<br>IR $\nu_{max}^{nujol}$ $cm^{-1}$: 3340, 3200, 1760, 1650, 1600<br>MS (m/z): 783 ($MNa^+$), 761 ($MH^+$)<br>NMR ($D_2O$) δ: 2.91 and 2.94 (1H, dx2, J=18Hz and 18Hz), 3.33 and 3.37 (1H, dx2, J=18Hz and 18Hz), 3.90, 4.28 and 3.98 (2H, ABq, J=14Hz and br s), 4.89 and 4.91 (1H, dx2, J=4.4Hz and 4.4Hz), 5.48 and 5.56 (1H, dx2, J=4.4Hz and 4.4Hz), 6.00 (1H, s), 6.67 and 6.75 (1H, dx2, J=9.8Hz and 9.8Hz), 6.92 and 6.99 (1H, dx2, J=7.8Hz and 8.3Hz), 7.02 and 7.04 (1H, sx2), 7.16 and 7.25 (1H, dx2, J=8.3Hz and 7.8Hz), 8.28 and 8.36 (1H, dx2, J=9.8Hz and 9.8Hz), 8.59 and 8.67 (1H, sx2) |
| 33 | 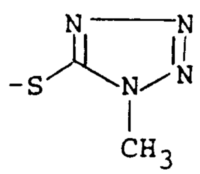 | M.p. 195-205°C (decomposed)<br>IR $\nu_{max}^{nujol}$ $cm^{-1}$: 3300, 3200, 1760, 1660, 1600<br>MS (m/z): 781 ($MNa^+$), 759 ($MH^+$)<br>NMR ($D_2O$) δ: 2.95 and 2.97 (1H, dx2, J=18Hz and 18Hz), 3.44 (1H, br d, J=18Hz), 3.97 and 4.01 (1H, dx2, J=14Hz and 13Hz), 4.02 (3H, s), 4.18 and 4.28 (1H, dx2, J=13Hz and 14Hz), 4.87 and 4.88 (1H, dx2, J=4.9Hz and 4.4Hz), 5.50 and 5.60 (1H, dx2, J=4.9Hz and 4.4Hz), 6.00 (1H, s), 6.77 (1H, d, J=9.8Hz), 7.02 and 7.03 (1H, sx2), 7.06 and 7.08 (1H, dx2, J=7.8Hz and 7.8Hz), 7.22 and 7.26 (1H, dx2, J=7.8Hz and 7.8Hz), 8.28 and 8.37 (1H, dx2, J=9.8Hz and 9.8Hz) |

*: The isomers are isolated by high performance liquid chromatography [solid phase: $C_{18}$-silica gel (YMC-Pack D-ODS-10), eluent: acetonitril/30 mM phosphate buffer (pH 7.0) = 6 : 94].

Example 34

A mixture of 4-(7-amino-3-methyl-3-cephem-4 carboxymethyl)anisole (502 mg), 2-(2-tritylaminothiazol-4-yl)-2-[(8-diphenylmethyloxy-2-oxo-1H-quinolin-5-yl)(diphenylmethyloxycarbonyl)methyloxyimino]acetic acid (979 mg), dicyclohexylcarbodiimide (227 mg), butyl alcohol (149 mg) and tetrahydrofuran (15 ml) is stirred at room temperature for 18 hours. The precipitates are removed by filtration, and the filtrate is concentrated, and the residue is purified by silica gel column chromatography (eluent; toluene/ethyl acetate = 1 : 1). The resulting foamy product is dissolved in anisole (5 ml), and thereto is added dropwise trifluoroacetic acid (5 ml) under ice-cooling, and the mixture is stirred at room temperature for two hours. To the reaction mixture is added a mixture of isopropyl ether (100 ml) and hexane (30 ml), and the precipitates are collected by

filtration, washed with isopropyl ether, dried, and dissolved in water. The pH value of the mixture is adjusted with saturated aqueous sodium hydrogen carbonate solution to about pH 8.0, and purified by HP-20 column chromatography (eluent; water and 20 % aqueous methanol). The fractions containing the desired compound are combined, concentrated, and lyophilized to give 7$\beta$-{(Z)-2-(2-aminothiazol-4-yl)-2-[(8-hydroxy-2-oxo-1H-quinolin-5-yl)(carboxy)methyoxyimino]acetamido}-3-methyl-3-cephem-4-carboxylic acid disodium salt (400 mg) as light yellow powder.

M.p. 185 - 210 °C (decomposed)

IR (Nujol): 3320, 3200, 1755, 1650, 1600 $cm^{-1}$

MS (m/z): 645 ($MH^+$)

NMR ($D_2O$) $\delta$: 1.87 and 1.88 (3H, sx2), 2.74 and 2.76 (1H, dx2, J = 18Hz), 3.30 (1H, dx2, J = 18Hz), 4.87 and 4.88 (1H, dx2, J = 4.5Hz), 5.49 and 5.58 (1H, dx2, J = 4.5Hz), 5.98 and 5.99 (1H, sx2), 6.76 and 6.77 (1H, dx2, J = 9.8Hz), 7.02 and 7.03 (1H, sx2), 7.04 and 7.05 (1H, dx2, J = 8Hz), 7.22 and 7.24 (1H, dx2, J = 8Hz), 8.29 and 8.37 (1H, dx2, J = 9.8Hz)

Examples 35 - 36

The corresponding starting compounds and 2-(2-tritylaminothiazol-4-yl)-2-[(8-diphenylmethyloxy-2-oxo-1H-quinolin-5-yl)(diphenylmethyloxycarbonyl)methyloxyimino]acetic acid are treated in the same manner as in Example 34 to give the compounds of the following Table 3.

Table 3

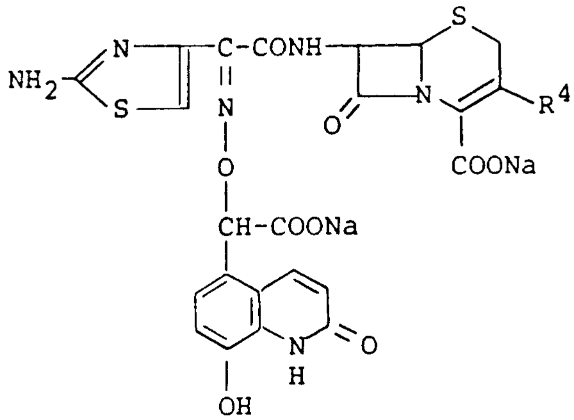

| Ex. No. | Compound | |
|---|---|---|
| | $R^4$ | Physicochemical properties |
| 35 | H | M.p. 165-175°C (decomposed)<br>IR $\nu_{max}^{nujol}$ $cm^{-1}$: 3320, 3200, 1760, 1650, 1605<br>MS (m/z): 630 ($MH^+$)<br>NMR ($D_2O$) δ: 2.95 and 3.01 (1H, ddx2, J=18Hz and 7Hz), 3.37 and 3.40 (1H, ddx2, J=18Hz and 2.5Hz), 4.89 and 4.90 (1H, dx2, J=5Hz), 5.60 and 5.69 (1H, dx2, J=5Hz), 5.99 (1H, s), 6.22 (1H, m), 6.75 and 6.77 (1H, dx2, J=10Hz), 7.02 and 7.03 (1H, sx2), 7.04 and 7.05 (1H, dx2, J=8Hz), 7.22 and 7.25 (1H, dx2, J=8Hz), 8.28 and 8.36 (1H, dx2, J=10Hz) |
| 36 | $-CH=CH_2$ | M.p. 180-187°C (decomposed)<br>IR $\nu_{max}^{nujol}$ $cm^{-1}$: 3340, 3200, 1760, 1650, 1605<br>MS (m/z): 657 ($MH^+$)<br>NMR ($D_2O$) δ: 3.16 and 3.18 (1H, dx2, J=18Hz), 3.32 and 3.34 (1H, dx2, J=18Hz), 4.93 and 4.95 (1H, dx2, J=5Hz), 5.27 (1H, dx2, J=11Hz), 5.40 (1H, dx2, J=17.5Hz), 5.54 and 5.63 (1H, dx2, J=5Hz), 5.99 and 6.00 (1H, sx2), 6.77 (1H, d, J=10Hz), 6.8 (1H, m), 7.02 and 7.04 (1H, sx2), 7.04 and 7.08 (1H, dx2, J=8Hz), 7.23 and 7.26 (1H, dx2, J=8Hz), 8.30 and 8.38 (1H, dx2, J=10Hz) |

Example 37

(1) A mixture of 5-oxalo-8-benzyloxycarbostyril (3.23 g), wet 10 % palladium-carbon (4 g, moisture: 50 %), and a mixture of tetrahydrofuran (100 ml), water (50 ml) and acetic acid (50 ml) is shaken at 45 °C for 40 hours under 4 atoms of hydrogen, and then the catalyst is removed by filtration. The catalyst is

washed with aqueous tetrahydrofuran, and the washings and the filtrate are combined and evaporated to remove the solvent therefrom. The residue is dissolved in 50 % aqueous tetrahydrofuran (200 ml), and thereto is added diphenyldiazomethane (2.9 g), and the mixture is stirred at room temperature for 20 hours. The solvent is distilled off, and to the resulting residue is added ether to break up the solid, and the solid is collected by filtration, and washed with ether, and dried under reduced pressure. The resulting solid is dissolved in dimethylformamide (20 ml), and thereto is added potassium carbonate (2.6 g). To the mixture is added dropwise a solution of benzhydril bromide (4.64 g) in dimethylformamide (5 ml), and the mixture is stirred at room temperature for five hours. The reaction solution is poured into ice-water (100 ml), and extracted with chloroform. The extract is washed successively with water and saturated saline solution, and dried. The solvent is distilled off, and the resulting residue is purified by silica gel column chromatography (eluent; hexane/ethyl acetate) to give 2-(8-diphenylmethyloxy-2-oxo-1,2,3,4-tetrahydroquinolin-5-yl)-2-hydroxyacetic acid diphenylmethyl ester (2.85 g, 50 %) as colorless crystals.

M.p. 165 - 166 °C
IR (Nujol): 3250, 1750, 1670, 1610, 1590 $cm^{-1}$
MS (m/z): 570 ($MH^+$)

(2) The product obtained above is treated in the same manner as in Example 1, (1)-(a) to give 2-(8-diphenylmethyloxy-2-oxo-1,2,3,4-tetrahydroquinolin-5-yl)-2-(phthalimidoxy)acetic acid diphenylmethyl ester.

M.p. 180 - 183 °C
IR (Nujol): 3240, 1790, 1760, 1740, 1670, 1610, 1590 $cm^{-1}$
MS (m/z): 715 ($MH^+$)

(3) The product obtained above is treated in the same manner as in Example 1-(2) to give 2-aminooxy-2-(8-diphenylmethyloxy-2-oxo-1,2,3,4-tetrahydroquinolin-5-yl)acetic acid diphenylmethyl ester.

M.p. 166 - 168 °C
IR (Nujol): 3410, 3320, 3240, 1750, 1680, 1610, 1590 $cm^{-1}$
MS (m/z): 585 ($MH^+$)

(4) The product obtained above is treated in the same manner as in Example 1-(3) to give 2-(2-tritylaminothiazol-4-yl)-2-[(8-diphenylmethyloxy-2-oxo-1,2,3,4-tetrahydroquinolin-5-yl)-(diphenylmethyloxycarbonyl)methyloxyimino]acetic acid.

M.p. 142 - 144 °C (decomposed)
IR (Nujol): 3400, 3220, 1740, 1700 (sh), 1680 $cm^{-1}$
MS (m/z): 981 ($MH^+$)

(5) The product obtained above is treated in the same manner as in Example 1-(4) to give 7β-{(Z)-2-(2-tritylaminothiazol-4-yl)-2-[(8-diphenylmethyloxy-2-oxo-1,2,3,4-tetrahydroquinolin-5-yl)-(diphenylmethyloxycarbonyl)methyloxyimino]acetamide}cephalosporanic acid.

M.p. 138 - 142 °C (decomposed)
IR (Nujol): 3400, 3250, 1790, 1740, 1690 $cm^{-1}$
MS (m/z): 1235 ($MH^+$)

Example 38

The corresponding starting compounds are treated in the same manner as in Example 2 to give 7β-{-(Z)-2-(2-aminothiazol-4-yl)-2-[(8-hydroxy-2-oxo-1,2,3,4-tetrahydroquinolin-5-yl)(carboxy)methyloxyimino]-acetamido}cephalosporanic acid.

M.p. >170 °C (decomposed)
IR (Nujol): 3300 (br), 3200 (sh), 1780, 1730, 1670 (br) $cm^{-1}$
MS (m/z): 661 ($MH^+$)

Disodium salt of this product

M.p. >170 °C (decomposed)
IR (Nujol): 3340 (br), 3200 (sh), 1760, 1660, 1600 $cm^{-1}$
MS (m/z): 727 ($MNa^+$), 705 ($MH^+$)

NMR ($D_2O$) δ: 2.13 and 2.15 (3H, sx2), 2.61-2.68 (2H, m), 3.01-3.13 (2H, m), 2.96, 3.46 (2H, ABq, J = 18Hz), 4.68 and 4.71 (1H, dx2, J = 13Hz and 12Hz), 4.77 and 4.89 (1H, dx2, J = 13Hz and 12Hz), 5.01 and 5.03 (1H, dx2, J = 4.4Hz and 4.4Hz), 5.64 and 5.68 (1H, dx2, J = 4.4Hz and 4.4Hz), 5.69 and 5.70 (1H, s), 6.82 and 6.83 (1H, dx2, J = 8.3Hz and 8.3Hz), 6.96 and 6.99 (1H, dx2, J = 8.3Hz and 8.3Hz), 7.02 (1H, s)

Example 39

(1) To a suspension of 2-(8-hydroxy-2-oxo-1H-quinolin-5-yl)-2-hydroxyacetic acid (11.8 g) in 17 % aqueous tetrahydrofuran (480 ml) is added diphenyldiazomethane (15.6 g), and the mixture is stirred at room temperature for three days. The solvent is distilled off, and to the resulting residue is added diethyl ether (250 ml) in order to crash the solid, and the solid is collected by filtration. The solid is washed with diethyl ether (300 ml) and dried under reduced pressure to give 2-(8-hydroxy-2-oxo-1H-quinolin-5-yl)-2-hydroxyacetic acid diphenylmethyl ester (17.16 g).

M.p. 218.5 - 219.5 °C (decomposed)
IR (Nujol): 3365, 3240, 2720-2580, 1730, 1645, 1630, 1590 $cm^{-1}$
MS (m/z): 402 ($MH^+$)

(2) To a solution of the product (16.15 g) obtained above in dimethylformamide (81 ml) are added successively potassium carbonate (4.2 g) and methyl iodide (7.8 ml), and the mixture is stirred at room temperature for 7 hours. To the mixture is added ice-water (480 ml) under ice-cooling, and the mixture is stirred. The precipitates are collected by filtration, washed with water, and dried under reduced pressure to give 2-(8-methoxy-2-oxo-1H-quinolin-5-yl)-2-hydroxyacetic acid diphenylmethyl ester (16.05 g).

M.p. 177 - 182 °C
IR (Nujol): 3330, 3160, 1740, 1650, 1630, 1600 $cm^{-1}$
MS (m/z): 416 ($MH^+$)

(3) The product obtained above is treated in the same manner as in Example 1, (1)-(a) to give 2-(8-methoxy-2-oxo-1H-quinolin-5-yl)-2-(phthalimidoxy)acetic acid diphenylmethyl ester.

M.p. 206 - 209 °C
IR (Nujol): 3170, 1790, 1740, 1660, 1610 $cm^{-1}$
MS (m/z): 561 ($MH^+$)

(4) The corresponding starting compounds are treated in the same manner as in Example 1-(2) to give 2-aminooxy-2-(8-methoxy-2-oxo-1H-quinolin-5-yl)acetic acid diphenylmethyl ester.

M.p. 166 - 168 °C
IR (Nujol): 3280, 3230, 3150, 1750, 1660, 1610 $cm^{-1}$
MS (m/z): 431 ($MH^+$)

(5) The corresponding starting compounds are treated in the same manner as in Example 1-(3) to give 2-(2-tritylaminothiazol-4-yl)-2-[(8-methoxy-2-oxo-1H-quinolin-5-yl)(diphenylmethyloxycarbonyl)-methyloxyimino]acetic acid.

M.p. 157 - 158.5 °C (decomposed)
IR (Nujol): 3380, 3220 (br), 1740, 1670, 1610 $cm^{-1}$
MS (m/z): 827 ($MH^+$)

(6) The corresponding starting compounds are treated in the same manner as in Example 1-(4) to give 7β-{(Z)-2-(2-tritylaminothiazol-4-yl)-2-[(8-methoxy-2-oxo-1H-quinolin-5-yl)(diphenylmethyloxycarbonyl)-methyloxyimino]acetamido}cephalosporanic acid.

M.p. 154 - 156 °C
IR (Nujol): 3220 (br), 1790, 1740, 1660, 1610 $cm^{-1}$
MS (m/z): 1081 ($MH^+$)

Example 40

The corresponding starting compounds are treated in the same manner as in Example 2 to give 7β-{-(Z)-2-(2-amino-thiazol-4-yl)-2-[(8-methoxy-2-oxo-1H-quinolin-5-yl)(carboxy)methyloxyimino]-acetamido}cephalosporanic acid.

M.p. >160 °C (decomposed)
IR (Nujol): 3300 (br), 3200 (br), 1780, 1730, 1660 (br), 1610 $cm^{-1}$
MS (m/z): 673 ($MH^+$)

Disodium salt of this product

M.p. >160 °C (decomposed)
IR (Nujol): 3350 (br), 3200 (sh), 1770, 1730 (sh), 1660, 1610 $cm^{-1}$
MS (m/z): 717($MH^+$)
NMR ($D_2O$) δ: 2.12 and 2.13 (3H, sx2), 2.87 and 2.93 (1H, dx2, J = 18Hz and 18Hz), 3.38 and 3.41 (1H, dx2, J = 18Hz and 18Hz), 4.01 and 4.02 (3H, sx2), 4.68 and 4.69 (1H, dx2, J = 13Hz and 13Hz), 4.85 and

4.87 (1H, dx2, J = 13Hz and 13Hz), 4.93 and 4.95 (1H, dx2, J = 4.9Hz and 4.9Hz), 5.60 and 5.68 (1H, dx2, J = 4.9Hz and 4.9Hz), 5.99 (1H, s), 6.77 and 6.79 (1H, dx2, J = 9.8Hz and 9.8Hz), 7.02 and 7.03 (1H, sx2), 7.18 and 7.20 (1H, dx2, J = 8.3Hz and 8.3Hz), 7.34 and 7.36 (1H, dx2, J = 8.3Hz and 8.3Hz), 8.29 and 8.37 (1H, dx2, J = 9.8Hz and 9.8Hz)

Example 41

(1) To a suspension of sodium hydride (750 mg) in dimethylformamide (50 ml) is added dropwise a solution of 2-(8-diphenylmethyloxy-2-oxo-1H-quinolin-5-yl)-2-(phthalimidoxy)acetic acid diphenylmethyl ester (10.6 g) in dimethylformamide (60 ml) over a period of 25 minutes under argon atmosphere, and the mixture is stirred at room temperature for 1.5 hour. The reaction solution is cooled to 0°C, and thereto is added dropwise methyl iodide (8.44 g), and the mixture is stirred at the same temperature for 1.5 hour. The mixture is poured into 0.05N-hydrochloric acid (330 ml), and the precipitates are extracted with ethyl acetate. The extract is washed with water, dried, and evaporated to remove the solvent therefrom. The resulting residue is purified by silica gel column chromatography (eluent; hexane/ethyl acetate) to give 2-(8-diphenylmethyloxy-1-methyl-2-oxo-1H-quinolin-5-yl)-2-(phthalimidoxy)acetic acid diphenylmethyl ester (5.28 g).

IR (Nujol) : 1790, 1760, 1740, 1660, 1610, 1590 $cm^{-1}$

MS (m/z): 727 ($MH^+$)

NMR ($CDCl_3$) $\delta$: 3.89 (3H, s), 6.21 (1H, s), 6.41 (1H, s), 6.70 (1H, d, J = 9.8Hz), 6.82-7.40 (23H, m), 7.69-7.80 (4H, m), 8.34 (1H, d, J = 9.8Hz)

(2) The product obtained above is treated in the same manner as in Example 1, (1)-(a) to give 2-aminooxy-2-(8-diphenylmethyloxy-1-mettiyl-2-oxo-1H-quinolin-5-yl)acetic acid diphenylmethyl ester.

IR (Nujol): 3310, 3240, 1750, 1660, 1610, 1590, 1560 $cm^{-1}$

MS (m/z): 597 ($MH^+$)

NMR ($CDCl_3$) $\delta$: 3.88 (3H, s), 5.55 (1H, s), 5.91 (2H, brs), 6.20 (1H, s), 6.61 (1H, d, J = 9.9Hz), 6.79-7.43 (23H, m), 8.00 (1H, d, J = 9.9Hz)

(3) The product obtained above is treated in the same manner as in Example 1-(2) to give 2-(2-tritylaminothiazol-4-yl)-2-[(8-diphenylmethyloxy-1-methyl-2-oxo-1H-quinolin-5-yl)-(diphenylmethyloxycarbonyl)methyloxyimino]acetic acid.

M.p. 140 - 145°C (decomposed)

IR (Nujol): 3220 (br), 1740, 1660, 1610 (sh), 1590 $cm^{-1}$

MS (m/z): 993 ($MH^+$)

NMR ($CDCl_3$) $\delta$:3.83 (3H, s), 6.16 (1H, s), 6.19 (1H, s), 6.52 (1H, d, J = 9.8Hz), 6.70 (1H, s), 6.79-7.36 (38H, m), 7.50 (1H, br), 8.02 (1H, d, J = 9.8Hz)

(4) The product obtained above is treated in the same manner as in Example 1-(3) to give 7$\beta$-{(Z)-2-(2-tritylaminothiazol-4-yl)-2-[(8-diphenylmethyloxy-1-methyl-2-oxo-1H-quinolin-5-yl)-(diphenylmethyloxycarbonyl)methyloxyimino]acetamido}cephalosporanic acid.

M.p. 139 - 141°C (decomposed)

IR (Nujol): 3240 (br), 1790, 1740, 1680, 1660 $cm^{-1}$

MS (m/z): 1247 ($MH^+$)

NMR (DMSO-$d_6$) $\delta$: 2.00 and 2.03 (3H, sx2), 3.00-3.60 (2H, m), 3.86 (3H, s), 4.65 and 4.68 (1H, dx2, J = 13Hz and 13Hz), 4.90 and 4.98 (1H, dx2, J = 13Hz and 13Hz), 4.94 and 5.08 (1H, dx2, J = 4.9Hz and 4.9Hz), 5.64 (1H, m), 6.18 (1H, s), 6.50 and 6.51 (1H, dx2, J = 9.8Hz and 9.8Hz), 6.67, 6.75, 6.78, 6.80 and 6.82 (3H, each s), 7.03-7.57 (37H, m), 8.08 and 8.10 (1H, dx2, J = 9.8Hz and 9.8Hz), 8.87 and 8.90 (1H, sx2), 9.59 and 9.63 (1H, dx2, J = 7.8Hz and 7.8 Hz)

Example 42

The compound obtained in Example 41 is treated in the same manner as in Example 2 to give 7$\beta$-{(Z)-2-(2-aminothiazol-4-yl)-2-[(8-hydroxy-1-methyl-2-oxo-1H-quinolin-5-yl)(carboxy)methyloxyimino]-acetamido}cephalosporanic acid.

M.p. >170°C (decomposed)

IR (Nujol): 3300 - 3200, 1780, 1740, 1650, 1590 $cm^{-1}$

Disodium salt of this product

M.p. >145 ° C (decomposed)
IR (Nujol): 3340 (br), 3200, 1770, 1650, 1590 $cm^{-1}$
NMR ($D_2O$) $\delta$: 2.13 and 2.14 (3H, sx2), 2.87 and 2.94 (1H, dx2, J=18Hz and 18Hz), 3.37 and 3.40 (1H, dx2, J=18Hz and 18Hz), 3.96 and 3.99 (3H, sx2), 4.69 (1H, brd, J=11Hz), 4.79-4.93 (1H, m), 4.87 and 4.91 (1H, dx2, J=4.9Hz and 4.9Hz), 5.56 and 5.66 (1H, dx2, J=4.9Hz and 4.9Hz), 5.99 (1H, s), 6.76 and 6.78 (1H, dx2, J=9.8Hz and 9.8Hz), 7.02 and 7.03 (1H, sx2), 7.14 and 7.15 (1H, dx2, J=8.3Hz and 8.3Hz), 7.25 and 7.28 (1H, dx2, J=8.3Hz and 8.3Hz), 8.16 and 8.25 (1H, dx2, J=9.8Hz and 9.8Hz)

Examples 43 - 48

The corresponding starting compounds are treated in the same manner as in Example 3 to give the compounds of the following Table 4.

Table 4

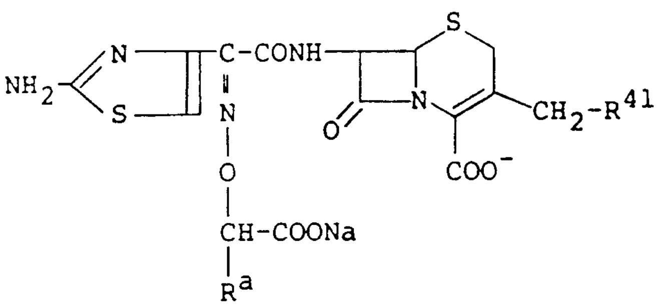

| Ex. No. | Compound | | |
|---|---|---|---|
| | $R^a$ | $R^{41}$ | Physicochemical properties |
| 43 | 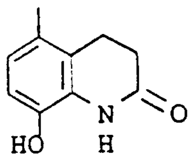 | 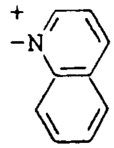 | M.p. >160°C (decomposed)<br>IR (Nujol): 3300(br), 3200(sh), 1770, 1660, 1610, 1590 $cm^{-1}$<br>NMR ($D_2O$) δ: 2.45-2.66 (3H, m), 2.75-3.07 (2H, m), 3.23 and 3.24 (1H, dx2, J=18Hz and 18Hz), 4.99 and 5.04 (1H, dx2, J=4.9Hz and 4.9Hz), 5.65 (1H, s), 5.67 and 5.70 (1H, dx2, J=4.9Hz and 4.9Hz), 5.78 and 5.79 (1H, dx2, J=15Hz and 15Hz), 5.92 (1H, d, J=15Hz), 6.43 and 6.62 (1H, dx2, J=8.3Hz and 8.8Hz), 6.85 and 6.91 (1H, dx2, J=8.8Hz and 8.3Hz), 6.95 and 6.96 (1H, sx2), 7.97-8.47 (5H, m), 9.15-9.27 (2H, m) |
| 44 | 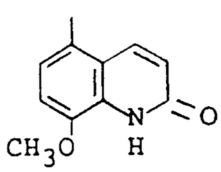 | 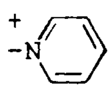 | M.p. >165°C (decomposed)<br>IR (Nujol): 3380(br), 3200(sh), 1780, 1660, 1610 $cm^{-1}$<br>MS (m/z): 736 ($MNa^+$), 714 ($MH^+$)<br>NMR ($D_2O$) δ: 2.63 and 2.72 (1H, dx2, J=18Hz and 18Hz), 3.39 and 3.48 (1H, dx2, J=18Hz and 18Hz), 3.86 and 3.93 (3H, sx2), 5.01 and 5.04 (1H, dx2, J=4.9Hz and 4.9Hz), 5.27 and 5.30 (1H, dx2, J=15Hz and 15Hz), 5.53 and 5.55 (1H, dx2, J=15Hz and 15Hz), 5.67 and 5.73 (1H, dx2, J=4.9Hz and 4.9Hz), 5.94 and 5.97 (1H, sx2), 6.69 and 6.70 (1H, dx2, J=9.8Hz and 9.8Hz), 6.95 and 7.10 (1H, dx2, J=8.3Hz and 8.3Hz), 6.98 (1H, s), 7.26 and 7.34 (1H, dx2, J=8.3Hz and 8.3Hz), 8.07-8.15 (2H, m), 8.25 and 8.33 (1H, dx2, J=9.8Hz and 9.8Hz), 8.61 (1H, m), 8.89-8.95 (2H, m) |

| | | | |
|---|---|---|---|
| 45 | 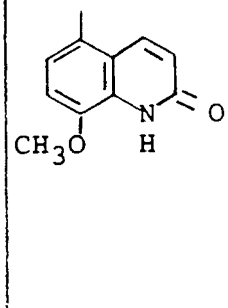 | 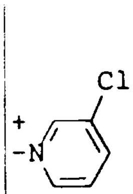 | M.p. >165°C (decomposed)<br>IR (Nujol): 3380(br), 3200(sh), 1770, 1660, 1610 $cm^{-1}$<br>MS (m/z): 726 ($MH^+$ free acid)<br>NMR ($D_2O$) δ: 2.69 and 2.78 (1H, dx2, J=18Hz and 18Hz), 3.43 and 3.52 (1H, dx2, J=18Hz and 18Hz), 3.88 and 3.93 (3H, sx2), 5.04 and 5.06 (1H, dx2, J=4.9Hz and 4.9Hz), 5.26 and 5.29 (1H, dx2, J=15Hz and 15Hz), 5.55 and 5.57 (1H, dx2, J=15Hz and 15Hz), 5.68 and 5.74 (1H, dx2, J=4.9Hz and 4.9Hz), 5.94 and 5.97 (1H, sx2), 6.69 and 6.71 (1H, dx2, J=9.8Hz and 9.8Hz), 6.96 and 6.97 (1H, sx2), 6.98 and 7.11 (1H, dx2, J=8.3Hz and 8.3Hz), 7.27 and 7.34 (1H, dx2, J=8.3Hz and 8.3Hz), 8.10 (1H, m), 8.26 and 8.34 (1H, dx2, J=9.8Hz and 9.8Hz), 8.63 (1H, m), 8.91 and 8.95 (1H, dx2, J=6.3Hz and 6.3Hz), 9.16 (1H, brs) |
| 46 | 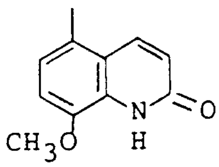 | 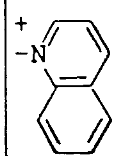 | M.p. >165°C (decomposed)<br>IR (Nujol): 3380(br), 3200(sh), 1770, 1660, 1610 $cm^{-1}$<br>MS (m/z): 786 ($MNa^+$), 764 ($MH^+$)<br>NMR ($D_2O$) δ: 2.62 and 2.66 (1H, dx2, J=18Hz and 18Hz), 3.14 and 3.17 (1H, dx2, J=18Hz and 18Hz), 3.68 an 3.78 (3H, sx2), 4.96 and 4.98 (1H, dx2, J=4.9Hz and 4.9Hz), 5.65 and 5.70 (1H, dx2, J=4.9Hz and 4.9Hz), 5.60-5.95 (2H, m), 5.91 and 5.93 (1H, sx2), 6.62 and 6.66 (1H, dx2, J=9.8Hz and 9.8Hz), 6.80 and 6.99 (1H, dx2, J=8.3Hz and 8.3Hz), 6.88 and 6.91 (1H, sx2), 7.21 and 7.30 (1H, dx2, J=8.3Hz and 8.3Hz), 7.87-8.40 (6H, m), 9.01-9.21 (2H, m) |

| | | | |
|---|---|---|---|
| 47 | 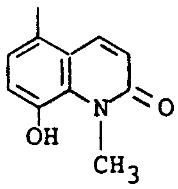 | 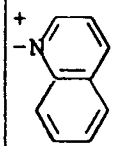 | M.p. >170°C (decomposed)<br>IR (Nujol): 3350(br), 3200(sh), 1770, 1650, 1590 $cm^{-1}$<br>MS (m/z): 786 ($MNa^+$), 764 ($MH^+$)<br>NMR ($D_2O$) δ: 2.56 and 2.65 (1H, dx2, J=18Hz and 18Hz), 3.13 and 3.15 (1H, dx2, J=18Hz and 18Hz), 3.46 and 3.55 (3H, sx2), 4.90 and 4.91 (1H, dx2, J=4.4Hz and 4.4Hz), 5.62 and 5.69 (1H, dx2, J=4.4Hz and 4.4Hz), 5.68-5.95 (2H, m), 5.91 and 5.92 (1H, sx2), 6.57 and 6.62 (1H, dx2, J=9.3Hz and 9.3Hz), 6.75 and 6.91 (1H, dx2, J=8.3Hz and 8.3Hz), 6.90 and 6.93 (1H, sx2), 7.12 and 7.21 (1H, dx2, J=8.3Hz and 8.3Hz), 7.82-8.38 (6H, m), 9.01 and 9.04 (1H, dx2, J=7.3Hz and 7.3Hz), 9.17 (1H, brd, J=5.8Hz) |
| 48 | α-Isomer*<br>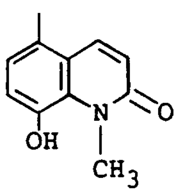 | 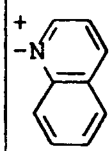 | M.p. >170°C (decomposed)<br>IR (Nujol): 3340(br), 3200, 1770, 1650, 1590 $cm^{-1}$<br>MS (m/z): 786 ($MNa^+$), 764 ($MH^+$)<br>NMR ($D_2O$) δ: 2.60, 3.13 (2H, ABq, J=18Hz), 3.58 (3H, s), 4.91 (1H, d, J=4.9Hz), 5.62 (1H, d, J=4.9Hz), 5.76, 5.90 (2H, ABq, J=16Hz), 5.92 (1H, s), 6.58 (1H, d, J=9.8Hz), 6.74 (1H, d, J=7.8Hz), 6.97 (1H, s), 7.11 (1H, d, J=7.8Hz), 7.90-8.39 (5H, m), 9.10 (1H, d, J=8.3Hz), 9.20 (1H, d, J=5.9Hz) |
| 48 | β-Isomer*<br>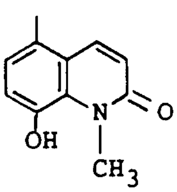 | 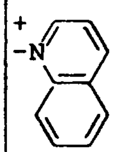 | M.p. >170°C (decomposed)<br>IR (Nujol): 3350(br), 3200, 1770, 1650, 1590 $cm^{-1}$<br>MS (m/z): 786 ($MNa^+$), 764 ($MH^+$)<br>NMR ($D_2O$) δ: 2.54, 3.12 (2H, ABq, J=18Hz), 3.48 (3H, s), 4.90 (1H, d, J=4.9Hz), 5.69 (1H, d, J=4.9Hz), 5.76, 5.88 (2H, ABq, J=16Hz), 5.93 (1H, s), 6.64 (1H, d, J=9.8Hz), 6.93 (1H, s), 6.94 (1H, d, J=8.3Hz), 7.21 (1H, d, J=8.3Hz), 7.95-8.05 (2H, m), 8.16-8.28 (3H, m), 8.39 (1H, d, J=8.8Hz), 9.07 (1H, d, J=8.3Hz), 9.19 (1H, d, J=5.4Hz) |

*: The isomers are isolated by high performance liquid chromatography [solid phase; $C_{18}$-silica gel (YMC-Pack D-ODS-10), eluent; acetonitril/30 mM phosphate buffer (pH 7.0) = 1 : 9].

Example 49

The compound obtained in Example 39 (disodium salt) and 1-allyl-4-thiopyridone are treated in the same manner as in Example 4 except that 50 % aqueous acetonitril is used as a solvent to give 7β-{(Z)-2-(2-aminothiazol-4-yl)-2-[(8-methoxy-2-oxo-1H-quinolin-5yl)(carboxy)methyloxyimino]acetamido}-3-(1-allyl-4-pyridiniothio)-3-cephem-4-carboxylic acid sodium salt.

M.p. >175 °C (decomposed)

IR (Nujol): 3370 (br), 3200 (sh), 1770, 1660, 1630, 1610 $cm^{-1}$

MS (m/z): 808 ($MNa^+$), 786 ($MH^+$)

NMR ($D_2O$) δ: 2.94 and 2.96 (1H, dx2, J=18Hz and 18Hz), 3.28 and 3.34 (1H, dx2, J=18Hz and 18Hz), 4.15 (1H, d, J=14Hz), 4.23 and 4.34 (1H, dx2, J=14Hz and 14Hz), 4.91-4.98 (3H, m), 5.38 (1H, d, J=17Hz), 5.49 (1H, d, J=10Hz), 5.56 and 5.61 (1H, dx2, J=4.9Hz and 4.9Hz), 5.92-6.12 (1H, m), 5.96 and 5.97 (1H, sx2), 6.71 and 6.74 (1H, dx2, J=9.8Hz and 9.8Hz), 6.95 (1H, s), 7.08 and 7.13 (1H, dx2, J=8.3Hz and 8.3Hz), 7.29 and 7.36 (1H, dx2, J=8.3Hz and 8.3Hz), 7.77 (2H, d, J=6.8Hz), 8.24-8.39 (3H, m)

Examples 50 - 52

7β-Amino-3-(3-amino-2-methyl-1-pyridiniomethyl)-3-cephem-4-carboxylate and the corresponding starting compounds are treated in the same manner as in Example 27 to give the compounds of the following Table 5.

Table 5

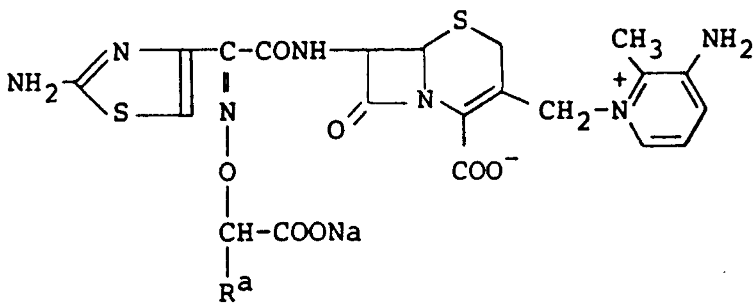

| Ex. No. | Compound | |
|---|---|---|
| | $R^a$ | Physicochemical properties |
| 50 | 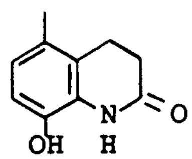 | M.p. >170°C (decomposed)<br>IR (Nujol): 3350, 3200, 1770, 1660, 1610 $cm^{-1}$<br>MS (m/z): 731 ($MH^+$)<br>NMR ($D_2O$) δ: 2.53 (3H, s), 2.46-2.73 (2H, m), 2.80-3.21 (2H, m), 5.00 and 5.05 (1H, dx2, J=4.9Hz and 4.9Hz), 5.24 and 5.28 (1H, dx2, J=15Hz and 15Hz), 5.50 (1H, brd), 5.65-5.70 (2H, m), 6.62 and 6.73 (1H, dx2, J=8.3Hz and 8.3Hz), 6.90 and 6.95 (1H, dx2, J=8.3Hz and 8.3Hz), 7.01 (1H, s), 7.61-7.54 (1H, m), 7.72 (1H, d, J=8.8Hz), 8.01 and 8.08 (1H, dx2, J=5.9Hz and 6.4Hz) |
| 51 | 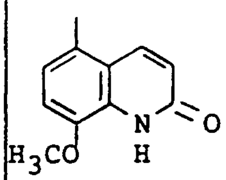 | M.p. >165°C (decomposed)<br>IR (Nujol): 3340(br), 3200(br), 1770, 1660, 1600 $cm^{-1}$<br>MS (m/z): 765 ($MNa^+$), 743 ($MH^+$)<br>NMR ($D_2O$) δ: 2.49 and 2.51 (3H, sx2), 2.54 and 2.60 (1H, dx2, J=18Hz and 18Hz), 3.06 and 3.10 (1H, dx2, J=18Hz and 18Hz), 3.85 and 3.91 (3H, sx2), 4.94 and 4.97 (1H, dx2, J=4.9Hz and 4.9Hz), 5.22 and 5.24 (1H, dx2, J=16Hz and 16Hz), 5.50 (1H, d, J=16Hz), 5.63-5.70 (1H, dx2, J=4.9Hz and 4.9Hz), 5.96 and 5.98 (1H, sx2), 6.70 and 6.74 (1H, dx2, J=9.8Hz and 9.8Hz), 6.98 and 6.99 (1H, sx2), 6.99 and 7.10 (1H, dx2, J=8.3Hz and 8.3Hz), 7.29 and 7.34 (1H, dx2, J=8.3Hz and 8.3Hz), 7.55 (1H, m), 7.67-7.69 (1H, dx2, J=8.3Hz and 8.3Hz), 7.99 (1H, m), 8.25 and 8.35 (1H, dx2, J=9.8Hz and 9.8Hz) |

| | | |
|---|---|---|
| 52 | 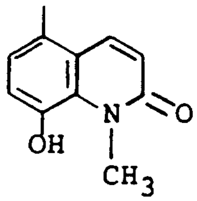 | M.p. >160°C (decomposed)<br>IR (Nujol): 3350, 3200, 1770, 1640, 1590 $cm^{-1}$<br>MS (m/z): 765 ($MNa^+$), 743 ($MH^+$)<br>NMR ($D_2O$) δ: 2.49 (3H, s), 2.56 and 2.65 (1H, dx2, J=18Hz and 18Hz), 3.02 and 3.04 (1H, dx2, J=18Hz and 18Hz), 3.75 and 3.77 (3H, sx2), 4.90 and 4.91 (1H, dx2, J=4.4Hz and 4.4Hz), 5.20 and 5.23 (1H, dx2, J=15Hz and 15Hz), 5.51 (1H, d, J=15Hz), 5.59 and 5.70 (1H, dx2, J=4.4Hz and 4.4Hz), 5.95 and 5.97 (1H, sx2), 6.66 and 6.71 (1H, dx2, J=9.8Hz and 9.8Hz), 6.93 and 7.04 (1H, dx2, J=8.3Hz and 8.3Hz), 6.98 (1H, s), 7.18 and 7.25 (1H, dx2, J=8.3Hz and 8.3Hz), 7.41-7.60 (1H, m), 7.65 and 7.67 (1H, dx2, J=8.3Hz and 8.3Hz), 7.96 and 7.99 (1H, dx2, J=5.9Hz and 5.9Hz), 8.08 and 8.22 (1H, dx2, J=9.8Hz and 9.8Hz) |
| 52 | α-Isomer*<br>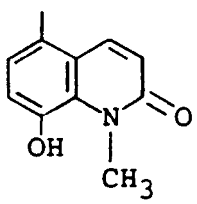 | M.p. >160°C (decomposed)<br>IR (Nujol): 3350, 3200, 1770, 1650, 1590 $cm^{-1}$<br>MS (m/z): 765 ($MNa^+$), 743 ($MH^+$)<br>NMR ($D_2O$) δ: 2.51 (3H, s), 2.56, 3.05 (2H, ABq, J=18Hz), 3.76 (3H, s), 4.92 (1H, d, J=4.4Hz), 5.25, 5.51 (2H, ABq, J=16Hz), 5.60 (1H, d, J=4.4Hz), 5.97 (1H, brs), 6.65 (1H, d, J=9.8Hz), 6.91 (1H, d, J=8.3Hz), 6.99 (1H, s), 7.18 (1H, d, J=8.3Hz), 7.58 (1H, dd, J=7.8Hz and 6.4Hz), 7.69 (1H, d, J=7.8Hz), 8.00 (1H, d, J=6.4Hz), 8.06 (1H, d, J=9.8Hz) |

| | | |
|---|---|---|
| 52 | β-Isomer* 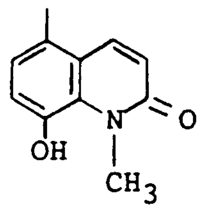 | M.p. >160°C (decomposed)<br>IR (Nujol): 3350, 3180, 1770, 1650, 1590 $cm^{-1}$<br>MS (m/z): 765 ($MNa^+$), 743 ($MH^+$)<br>NMR ($D_2O$) δ: 2.49 (3H, s), 2.55, 3.02 (2H, ABq, J=18Hz), 3.75 (3H, s), 4.90 (1H, d, J=4.9Hz), 5.21, 5.51 (2H, ABq, J=16Hz), 5.70 (1H, d, J=4.9Hz), 5.96 (1H, s), 6.71 (1H, d, J=9.8Hz), 6.98 (1H, s), 7.04 (1H, d, J=8.3Hz), 7.25 (1H, d, J=8.3Hz), 7.52 (1H, dd, J=8.3Hz, 5.9Hz), 7.66 (1H, d, J=8.3Hz), 7.97 (1H, d, J=5.9Hz), 8.23 (1H, d, J=9.8Hz) |

*: The isomers are isolated by high performance liquid chromatography [solid phase; $C_{18}$-silica gel (YMC-Pack D-ODS-10), eluent; acetonitril/30 mM phosphate buffer (pH 7.0) = 8 : 92].

Example 53

(1) 2-(8-Diphenylmethyloxy-2-oxo-1H-quinolin-5-yl)-2-hydroxyacetic acid diphenylmethyl ester (5.03 g) is dissolved in 17 % aqueous tetrahydrofuran (96 ml), and to the mixture is added dropwise 2.0N-aqueous sodium hydroxide solution (8.8 ml) with stirring under ice-cooling, and the mixture is stirred at room temperature for two hours. To the mixture is added dropwise 2.4N-hydrochloric acid (7.4 ml) under ice-cooling, and then added aqueous tetrahydrofuran (q.s.). The mixture is made homogeneous, and tetrahydrofuran is distilled off. The precipitates are collected by filtration, washed successively with water and diethyl ether, and dried under reduced pressure at 60 ° C to give 2-(8-diphenylmethyloxy-2-oxo-1H-quinolin-5-yl)-2-hydroxyacetic acid (3.42 g) as a colorless solid.

M.p. 186 - 188 ° C (decomposed)
IR (Nujol): 3360, 3160, 1710, 1640, 1600 $cm^{-1}$
MS (m/z): 401 ($M^+$)

(2) The product (145.8 g) obtained above is reacted with (R)-2-amino-3-methyl-1,1-diphenylbutan-1-ol (92.8 g), and the resulting salt is recrystallized twice from methanol to give optically active 2-(8-diphenylmethyloxy-2-oxo-1H-quinolin-5-yl)-2-hydroxyacetic acid (R)-2-amino-3-methyl-1,1-diphenylbutan-1-ol salt (47.7 g) as colorless crystals.

M.p. 208.5 - 209.5 ° C (decomposed)
IR (Nujol): 3440, 3400, 3360, 3260, 1660, 1630, 1600, 1590 $cm^{-1}$
MS (m/z): 657 ($MH^+$)
$[\alpha]_D^{20}$ -53.6 ° (c = 1.00, methanol)

(3) To a suspension of the product (111 g) obtained above in tetrahydrofuran (3.3 liters) is added diphenyldiazomethane (49.2 g), and the mixture is stirred at room temperature for two days. The resulting dark red-purple solution is evaporated to remove tetrahydrofuran therefrom, and the residue is subjected to azeotropic distillation with diethyl ether (200 ml) twice to give crystals. To the crystals is added diisopropyl ether (1 liter) in order to break up the crystals, and the crystals are collected by filtration, washed with diethyl ether (1 liter), and dried under reduced pressure at 45 ° C to give optically active 2-(8-diphenylmethyloxy-2-oxo-1H-quinolin-5-yl)-2-hydroxyacetic acid diphenylmethyl ester (94.6 g).

M.p. 194 - 196 ° C
IR (Nujol): 3320, 1740, 1640, 1600 $cm^{-1}$

MS (m/z): 568 ($MH^+$)

$[\alpha]_D^{20}$ -6.2° (c=1.00, chloroform)

Optical purity: >98 % ee [determined by high performance liquid chromatography (solid phase; cellulose derivative-coating silica column; tradename: Opti-Pak XC, eluent; hexane/ethanol = 3 : 2]

(4) The product obtained above is treated in the same manner as in Example 1, (1)-(a) to give optically active 2-(8-diphenylmethyloxy-2-oxo-1H-quinolin-5-yl)-2-(phthalimidoxy)acetic acid diphenylmethyl ester.

M.p. 194 - 196°C

IR (Nujol): 3180, 3060, 3020, 1790', 1760, 1740, 1650, 1610 $cm^{-1}$

MS (m/z): 713 ($MH^+$)

(5) The product obtained above is treated in the same manner as in Example 1-(2) to give optically active 2-aminohydroxy-2-(8-diphenylmethyloxy-2-oxo-1H-quinolin-5-yl)acetic acid diphenylmethyl ester.

M.p. 182 - 184°C (decomposed)

IR (Nujol): 3320, 3300, 3220, 3160, 1750, 1730, 1650, 1600 $cm^{-1}$

MS (m/z): 583 ($MH^+$)

(6) The product obtained above is treated in the same manner as in Example 1-(3) to give optically active 2-(2-tritylaminothiazol-4-yl)-2-[(8-diphenylmethyloxy-2-oxo-1H-quinolin-5-yl)(diphenylmethyloxycarbonyl)methyloxyimino]acetic acid.

M.p. 154 - 156°C

IR (Nujol): 3400, 3200 (br), 1740, 1670, 1610 $cm^{-1}$

MS (m/z): 979 ($MH^+$)

(7) The product obtained above is treated in the same manner as in Example 1-(4) to give optically active 7β-[(Z)-2-(2-tritylaminothiazol-4-yl)-2-[(8-diphenylmethyloxy-2-oxo-1H-quinolin-5-yl)-(diphenylmethyloxycarbonyl)methyloxyimino]acetamido}cephalosporanic acid.

M.p. 142 - 145°C (decomposed)

IR (Nujol): 3400, 3250 (br), 1790, 1740, 1670, 1600 $cm^{-1}$

MS (m/z): 1233 ($MH^+$)

NMR (DMSO-$d_6$) δ: 1.99 (3H, s), 3.09, 3.35 (2H, ABq, J=18Hz), 4.62, 4.91 (2H, ABq, J=13Hz), 4.94 (1H, d, J=4.9Hz), 5.62 (1H, dd, J=7.8Hz, 4.9Hz), 6.13 (1H, s), 6.46 (1H, d, 3=9.8Hz), 6.77-7.38, 7.72-7.76 (40H, m), 8.15 (1H, d, J=9.8Hz), 8.90 (1H, brs), 9.59 (1H, d, J=7.8Hz), 11.0 (1H, brs)

Examples 54 - 55

The optically active cephalosporanic acid compound obtained in Example 53-(7) and the corresponding starting compounds are treated in the same manner as in Example 3-(1) to give the following compounds.

(54) Optically active 7β-{(Z)-2-(2-aminothiazol-4-yl)-2-[(8-hydroxy-2-oxo-1H-quinolin-5-yl)(carboxy)-methyloxyimino]acetamido}-3-(1-quinoliniomethyl)-3-cephem-4-carboxylic acid sodium salt

The physicochemical properties of the above compound are the same as those of the β-isomer obtained in Example 3-(2).

(55) Optically active 7β-{(Z)-2-(2-aminothiazol-4-yl)-2-[(8-hydroxy-2-oxo-1H-quinolin-5-yl)(carboxy)methyloxy imino]acetamido}-3-(3-chloro-1-pyridiniomethy)-3-cephem-4-carboxylic acid sodium salt

The physicochemical properties of the above compound are the same as those of the β-isomer obtained in Example 26.

Example 56

The optically active oxyiminoacetic acid compound obtained in Example 53-(6) and the corresponding starting compounds are treated in the same manner as in Example 27-(1) to give optically active 7β-{(Z)-2-(2-aminothiazol-4-yl)-2-[(8-hydroxy2-oxo-1H-quinolin-5-yl)(carboxy)methyloxyimino]acetamido}-3-(3-amino-2-methyl-1-pyridiniomethyl)-3-cephem-4-carboxylic acid sodium salt.

The physicochemical properties of the above compound are the same as those of the β-isomer obtained in Example 27-(2).

Example 57

(1) To a solution of the compound (2.45 g) obtained in Example 53-(6) in tetrahydrofuran (20 ml) are added with ice-cooling 1-hydroxybenzotriazole (405 mg) and dicyclohexylcarbodiimide (567 mg) under argon atmosphere, and the mixture is stirred at room temperature for three hours (the resulting suspension is referred to as "Reaction solution A"). Separately, a solution of 7β-phenylacetamido-3-chloromethy1-3-cephem-4-carboxylic acid 4-methoxybenzyl ester (1.58 g) in dichloroethane (40 ml) is cooled with ice under argon atmosphere, and thereto are added pyridine (0.49 ml) and phosphorus pentachloride (1.01 g), and the mixture is stirred at the same temperature for one hour, and then at room temperature for 30 minutes. To the mixture is added dropwise methanol (24 ml) under ice-cooling, and the mixture is reacted at room temperature for 15 minutes. After cooling with ice, to the mixture is added 0.5N-aqueous potassium dihydrogen phosphate solution (48 ml), and the mixture is stirred at room temperature for 1.5 hour. The reaction solution is extracted with dichloromethane, and the extract is washed with saline solution, dried, and the solvent is distilled off. The resulting residue (1.87 g) is dissolved in tetrahydrofuran (15 ml) (the resulting solution is referred to as "Reaction solution B"). To Reaction solution A is added dropwise Reaction solution B, and the mixture is stirred at room temperature for 17 hours. The precipitates are removed by filtration, and the filtrate is evaporated to remove the solvent therefrom. The oily residue is purified by silica gel column chromatography (eluent; hexane/ethyl acetate) to give optically active 7β-{(Z)-2-(2-tritylaminothiazol-4-yl)-2-[(8-diphenylmethyloxy-2-oxo-1H-quinolin-5-yl)(diphenyloxymethylcarbony)methyloxyimino]acetamide}-3-(chloromethyl)-3-cephem-4-carboxylic acid 4-methoxybenzyl ester (2.12 g).

IR (Nujol): 3380, 3280, 3200, 1790, 1740, 1660, 1610 $cm^{-1}$

(2) To a solution of the product (800 mg) obtained above in dimethylacetamide (8 ml) are added with ice-cooling successively zinc iodide (77 mg) and 1-allyl-4-thiopyridone (272 mg) under argon atmosphere, and the mixture is stirred at the same temperature for 22 hours. To the reaction mixture are added water (20 ml) and saturated saline solution (20 ml) under ice-cooling, and the precipitates are collected by filtration, washed successively with water and ethyl acetate, and concentrated to dryness under reduced pressure at room temperature overnight. The resulting solid (840 mg) is added to a mixture of trifluoroacetic acid (2.5 ml)/anisole (2.5 ml) under ice-cooling, and the mixture is stirred at room temperature for two hours. The mixture is cooled with ice, and thereto is added diisopropyl ether (50 ml), and the mixture is stirred for 15 minutes. The precipitates are collected by filtration, washed with diisopropyl ether, and dried under reduced pressure at room temperature. The resulting solid (555 mg) is suspended in water (5 ml), and the pH value of the mixture is adjusted to pH 8.0 with saturated aqueous sodium hydrogen carbonate solution, and purified by HP-20 column chromatography (eluent; water and 20 % aqueous methanol). The fractions containing the desired compound are combined, concentrated and lyophilized to give optically active 7β-{(Z)-2-(2-aminothiazol-4-yl)-2-[(8-hydroxy-2-oxo-1H-quinolin-5-yl)-(carboxy)methyloxyimino]acetamido}-3-[(1-allyl-4-pyridinio)thiomethyl]-3-cephem-4-carboxylic acid sodium salt (104 mg).

M.p. >165 °C (decomposed)

IR (Nujol): 3350 (dr), 3180 (br), 1770, 1660, 1630, 1610 $cm^{-1}$

NMR (DMSO-$d_6$) δ: 2.96, 3.34 (2H, ABq, J=18Hz), 4.79 (2H, brs), 4.90-4.93 (3H, m), 5.33 (1H, d, J=17Hz), 5.47 (1H, d, J=9.8Hz), 5.90-6.10 (1H, m), 5.97 (1H, s), 6.74 (1H, d, J=9.8Hz), 6.95 (1H, s), 7.01 (1H, d, J=8.3Hz), 7.23 (1H, d, J=8.3Hz), 7.73 (2H, d, J=6.8Hz), 8.29 (2H, d, J=6.8Hz), 8.35 (1H, d, J=9.8Hz)

Example 58

To a solution of the compound (1.33 g) obtained in Example 57-(1) in acetonitril (13 ml) are added with ice-cooling sodium iodide (164 mg) and 3-chloropyridine (454 mg) under argon atmosphere, and the mixture is stirred at 8 °C for 14 hours. The mixture is evaporated under reduced pressure to remove acetonitril therefrom, and to the resulting residue is added a mixture of trifluoroacetic acid (8 ml)/anisole (8 ml) under ice-cooling, and the mixture is stirred at room temperature for two hours. The mixture is cooled with ice, and thereto is added diisopropyl ether (150 ml), and the mixture is stirred for 15 minutes. The precipitates are collected by filtration, washed with diisopropyl ether and dried under reduced pressure at room temperature. The resulting solid is suspended in water (20 ml), and the pH value thereof is adjusted to pH 8.0 with saturated aqueous sodium hydrogen carbonate solution, and purified HP-20 column chromatography (eluent; water and 20 % aqueous methanol). The fractions containing the desired compound are combined, concentrated and lyophilized to give optically active 7β-{(Z)-2-(2-aminothiazol-4-yl)-2-[(8-hydroxy-2-oxo-1H-quiolin-5-yl)(carboxy)methyloxyimino]acetamido}-3-(3-chloro-1-pyridiniomethyl)-3-cephem-4-carboxylic acid sodium salt (220 mg).

The physicochemical properties of the above compound are the same as those of the β-isomer obtained in Example 26.

[Preparation of the starting compounds]

Reference Example 1

(1) 5-(2,2-Dihydroxyacetyl)-8-(benzyloxy)-carbostyril (58 g) is suspended in tert-butanol (1000 ml) and 5 % aqueous potassium dihydrogen phosphate solution (650 ml), and to the suspension is added dropwise with stirring to a mixture of potassium permanganate (56.3 g ) and water (930 ml) at 5 - 7 ° C, and the mixture is stirred at 5 - 7 ° C for seven hours, and then at room temperature for 17 hours. To the mixture is added saturated aqueous sodium sulfite solution (535 ml) under ice-cooling, and the pH value thereof is adjusted to pH 1-2 with conc. hydrochloric acid, and the mixture is stirred for 30 minutes. The precipitates are collected by filtration, washed with water and dried to give 5-oxalo-8-(benzyloxy)-carbostyril (36.7 g).

M.p. 217 - 219 ° C (decomposed)

IR (Nujol): 3200 - 2400, 1700, 1670, 1640, 1600 $cm^{-1}$

MS (m/z): 323 ($M^+$)

(2) To a solution of the product (74 g) obtained above in tetrahydrofuran (2000 ml) and water (1500 ml) is added wet 10 % palladium-carbon (37 g, moisture: 50 %), and the mixture is stirred for three hours under hydrogen atmosphere, and the catalyst is separated by filtration. The catalyst is washed with a mixture of tetrahydrofuran and water, and the washings and the filtrate are combined, and evaporated to remove the solvent therefrom, and dried to give 2-hydroxy-2-(8-hydroxy-2-oxo-1H-quinolin-5-yl)acetic acid (54 g).

M.p. 259 - 261 ° C (decomposed)

IR (Nujol): 3160, 3080, 2780 - 2560, 1725, 1695, 1640, 1600 $cm^{-1}$

MS (m/z): 236 ($MH^+$)

(3) The product (39 g) obtained above is dissolved in tetrahydrofuran (3000 ml) and water (600 ml) with heating, and then cooled to 40 ° C. To the mixture is added diphenyldiazomethane (96.6 g), and the mixture is stirred for one hour, and then at 55 ° C for 20 hours. To the mixture is added additional diphenyldiazomethane (96.6 g), and the mixture is stirred for 24 hours. The solvent is distilled off, and to the residue is added ether to give crystals. The precipitated crystals are collected by filtration, washed with ether and dried to give 2-(8-diphenylmethyloxy2-oxo-1H-quinolin-5-yl)-2-hydroxyacetic acid diphenylmethyl ester (57.2 g).

M.p. 183 - 185 ° C (decomposed)

IR (Nujol) : 3330, 1750, 1650, 1600 $cm^{-1}$

MS (m/z): 568 ($MH^+$)

[Effects of the Invention]

The desired compound [I] of the present invention and pharmaceutically acceptable salts thereof have excellent antimicrobial activities against the wide range of various microorganisms including Gram positive bacteria and Gram negative bacteria, more particularly, have high stability to β-lactamase-producing bacteria, and hence, these compounds can be used as chemotherapeutics in the treatment of various infectious diseases caused by above mentioned microorganisms in mammals including human beings or additives for animal feeds.

## Claims

**1.** A cephalosporin compound of the formula [I]:

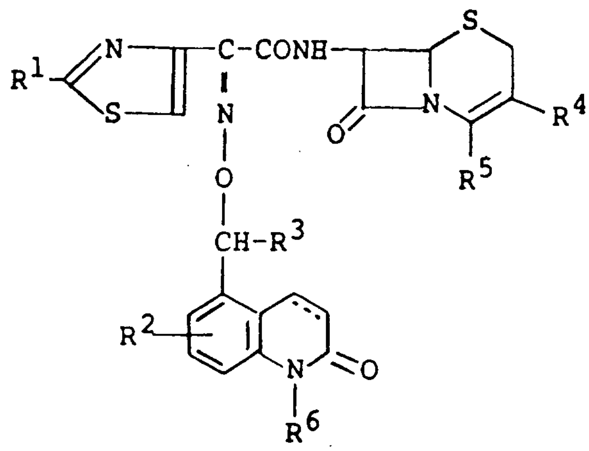

[I]

wherein $R^1$ is an amino group or a protected amino group, $R^2$ is a hydroxy group, a protected hydroxy group or a lower alkoxy group, $R^3$ is a carboxyl group or a protected carboxyl group, $R^4$ is hydrogen atom, a lower alkyl group, a lower alkenyl group or a group of the formula: $-CH_2R^{41}$, wherein $R^{41}$ is a nucleophilic residue, $R^5$ is a carboxyl group, a protected carboxyl group or a group of the formula: $-COO^-$, $R^6$ is a hydrogen atom or a lower alkyl group, and the broken line means the presence or absence of a double bond, or a pharmaceutically acceptable salt thereof.

**2.** A compound as claimed in claim 1, wherein $R^{41}$ is a lower alkanoyloxy group; a nitrogen-containing heterocyclic group having optionally substitutents; or a thio group substituted by a nitrogen-containing heterocyclic group having optionally a substituent.

**3.** A compound as claimed in claim 2, wherein $R^{41}$ is a lower alkanoyloxy group; pyridinio group, quinolinio group, isoquinolinio group, 2,3-propanopyridinio group, 5,6,7,8-tetrahydroquinolinio group or 2,3-pentanopyridinio group, which optionally have one or two substituents selected from the group consisting of a halogen atom, a lower alkyl group, a lower alkoxy group, a lower alkoxycarbonyl group, amino group, carbamoyl group, cyano group, formylamino group and hydroxy(lower)alkyl group; thiadiazolylthio group or tetrazolylthio group, which may optionally be substituted by a lower alkyl group; or a pyridiniothio group which may optionally be substituted by a lower alkenyl group.

**4.** A compound as claimed in claim 3, wherein $R^4$ is a hydrogen atom, methyl group, vinyl group or a group of the formula: $-CH_2R^{41}$, wherein $R^{41}$ is acetoxy group; pyridinio group, quinolinio group, isoquinolinio group, 2,3-propanopyridinio group, 5,6,7,8-tetrahydroquinolinio group or 2,3-pentanopyridinio group, which may have one or two substituents selected from the group consisting of a halogen atom, methyl group, methoxy group, methoxycarbonyl group, amino group, carbamoyl group, cyano group, formylamino group and a hydroxymethyl group; (1,2,3-thiadiazol-5-yl)thio group or 5-tetrazolylthio group, which may optionally be substituted by a methyl group; or 1-allyl-4-pyridiniothio group.

**5.** A compound as claimed in any one of the preceding claims wherein $R^1$ is an amino group, $R^3$ is a carboxyl group, $R^5$ is a carboxyl group or a group of the formula: $-COO^-$, and $R^6$ is a hydrogen atom or a methyl group.

**6.** A compound as claimed in claim 5, wherein $R^2$ is a hydroxy group or a methoxy group, which is substituted at the 8-position of the 2-oxo-1H-quinoline ring or the 2-oxo-1,2,3,4-tetrahydroquinoline ring.

**7.** A compound as claimed in claim 6, wherein $R^2$ is a hydroxy group, $R^6$ is a hydrogen atom and the broken line means the presence of a double bond.

**8.** A compound as claimed in claim 7, wherein $R^4$ is a group of the formula: $-CH_2R^{41}$ wherein $R^{41}$ is an acetoxy group, a pyridinio group, a 3-chloropyridinio group, a 3-amino-2-methylpyridinio group or a quinolinio group.

**9.** A compound as claimed in claim 8, wherein $R^{41}$ is a pyridinio group, a 3-chloropyridinio group, a 3-amino-2-methylpyridinio group or a quinolinio group.

**10.** 7β-{(Z)-2-(2-Aminothiazol-4-yl)-2-[(8-hydroxy-2-oxo-1H-quinolin-5-yl)(carboxy)methyloxyimino]-acetamido}-3-(1-quinoliniomethyl)-3-cephem-4-carboxylic acid, or a pharmaceutically acceptable salt thereof.

**11.** 7β-{(Z)-2-(2-Aminothiazol-4-yl)-2-[(8-hydroxy-2-oxo-1H-quinolin-5-yl)(carboxy)methyloxyimino]-acetamido}-3-(3-chloro-1-pyridiniomethyl)-3-cephem-4-carboxylic acid, or a pharmaceutically acceptable salt thereof.

**12.** 7β-{(Z)-2-(2-Aminothiazol-4-yl)-2-[(8-hydroxy-2-oxo-1H-quinolin-5-yl)(carboxy)methyloxyimino]-acetamido}-3-(3-amino-2-methyl-1-pyridiniomethyl)-3-cephem-4-carboxylic acid, or a pharmaceutically acceptable salt thereof.

**13.** A process for preparing the cephalosporin compound of the formula [I]:

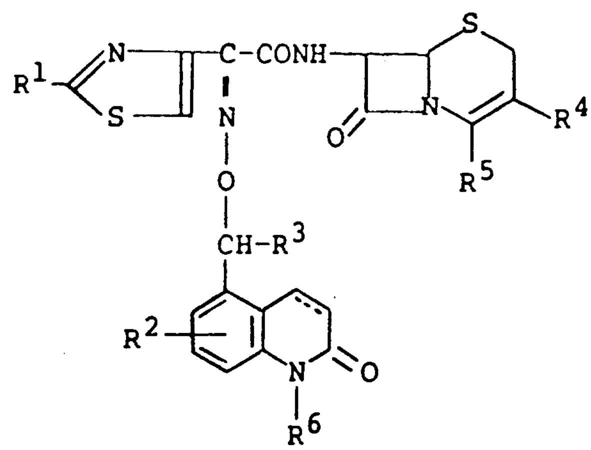

[I]

wherein $R^1$ is an amino group or a protected amino group, $R^2$ is a hydroxy group, a protected hydroxy group or a lower alkoxy group, $R^3$ is a carboxyl group or a protected carboxyl group, $R^4$ is a hydrogen atom, a lower alkyl group, a lower alkenyl group or a group of the formula: $-CH_2R^{41}$ wherein $R^{41}$ is a nucleophilic residue, $R^5$ is a carboxyl group, a protected carboxyl group or a group of the formula: $-COO^-$, $R^6$ is a hydrogen atom or a lower alkyl group, and the broken line means the presence or absence of a double bond, or a pharmaceutically acceptable salt thereof, which comprises condensing an oxyiminoacetic acid compound of the formula [II]:

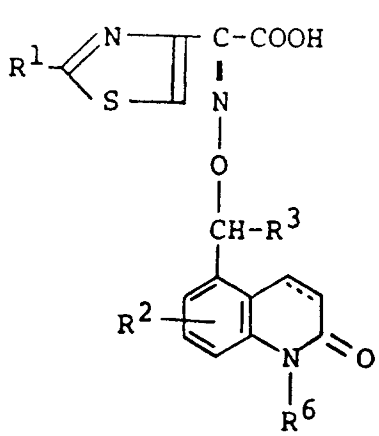

[II]

wherein $R^1$, $R^2$, $R^3$, $R^6$ and the broken line are the same as defined above, a salt or a reactive derivative thereof, with a 7-aminocephalosporin compound of the formula [III]:

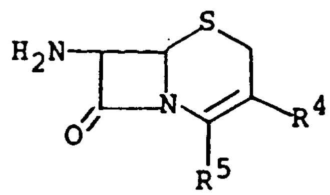

[III]

wherein $R^4$ and $R^5$ are the same as defined above or a salt thereof, optionally followed by converting the product into a pharmaceutically acceptable salt thereof.

**14.** A process for preparing the cephalosporin compound of the formula [I]:

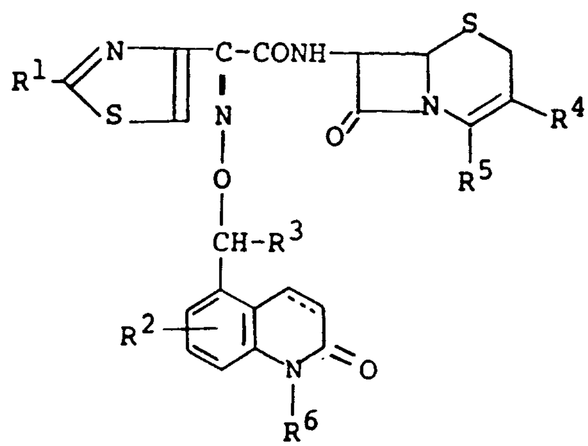

[I]

therein $R^1$ is an amino group or a protected amino group, $R^2$ is a hydroxy group, a protected hydroxy group or a lower alkoxy group, $R^3$ is a carboxyl group or a protected carboxyl group, $R^4$ is a hydrogen atom, a lower alkyl group, a lower alkenyl group or a group of the formula: $-CH_2R^{41}$ wherein $R^{41}$ is a nucleophilic residue, $R^5$ is a carboxyl group, a protected carboxyl group or a group of the formula: $-COO^-$, $R^6$ is a hydrogen atom or a lower alkyl group, and the broken line means the presence or absence of a double bond, or a pharmaceutically acceptable salt thereof, which comprises reacting a compound of the formula [IV]:

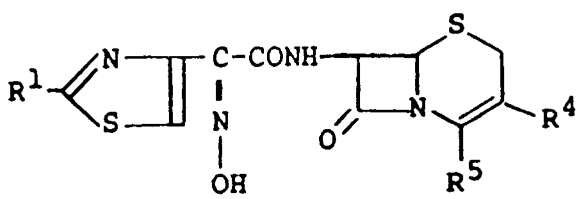

[IV]

wherein $R^1$, $R^4$ and $R^5$ are the same as defined above or a salt thereof, with a compound of the formula [V]:

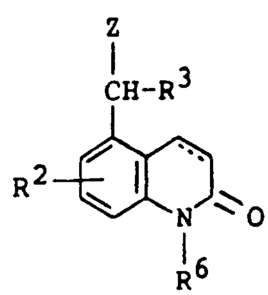

[V]

wherein Z is a reactive residue, $R^2$, $R^3$, $R^6$ and the broken line are the same as defined above, or a salt thereof, optionally followed by converting the product into a pharmaceutically acceptable salt thereof.

**15.** A process for preparing a cephalosporin compound of the formula [I-a]:

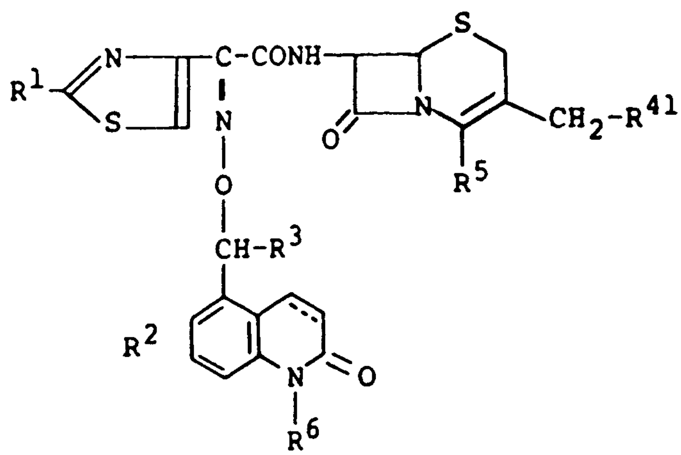

[I-a]

wherein $R^1$ is an amino group or a protected amino group, $R^2$ is a hydroxy group, a protected hydroxy group or a lower alkoxy group, $R^3$ is a carboxyl group or a protected carboxyl group, $R^{41}$ is a nucleophilic residue, $R^5$ is a carboxyl group, a protected carboxyl group or a group of the formula: $-COO^-$, $R^6$ is a hydrogen atom or a lower alkyl group, and the broken line means the presence or absence of a double bond, or a pharmaceutically acceptable salt thereof, which comprises reacting a cephalosporin compound of the formula [VI]:

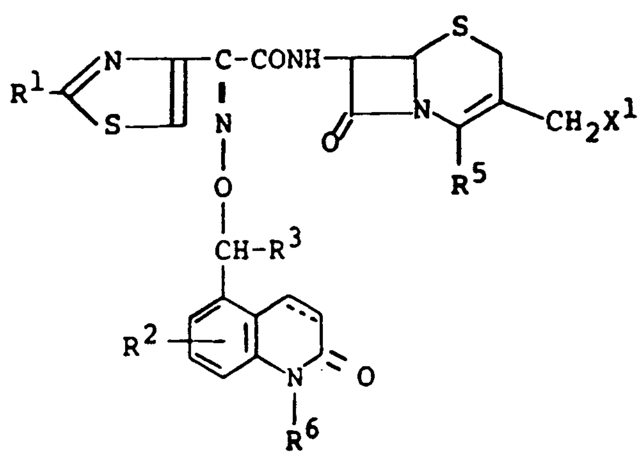

[VI]

wherein $X^1$ is a reactive residue, $R^1$, $R^2$, $R^3$, $R^5$, $R^6$ and the broken line are the same as defined above, or a salt thereof, with a nucleophilic compound or a salt thereof, optionally followed by converting the product into a pharmaceutically acceptable salt thereof.

**16.** A thiazolylacetic acid derivative of the formula [II-a]:

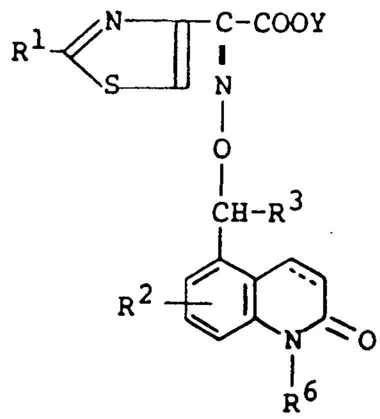

[II-a]

wherein $R^1$ is an amino group or a protected amino group, $R^2$ is a hydroxy group, a protected hydroxy group or a lower alkoxy group, $R^3$ is a carboxyl group or a protected carboxyl group, a group of the formula: -COOY where -COOY is a carboxyl group or a protected carboxyl group, $R^6$ is a hydrogen atom or a lower alkyl group and the broken line means the presence or absence of a double bond, or a salt thereof.

**17.** A carbostyril compound of the formula [V]:

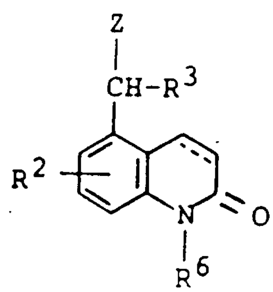

[V]

wherein $R^2$ is a hydroxy group or a protected hydroxy group, $R^3$ is a carboxyl group or a protected carboxyl group, $R^6$ is a hydrogen atom or a lower alkyl group, Z is a reactive residue and the broken line means the presence or absence of a double bond, or a salt thereof.

**18.** A cephalosporin compound of the formula [VI]:

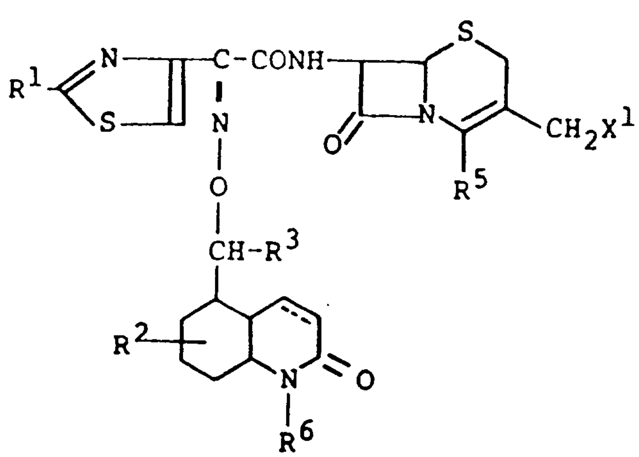

[VI]

wherein $R^1$ is an amino group or a protected amino group, $R^2$ is a hydroxy group, a protected hydroxy group or a lower alkoxy group, $R^3$ is a carboxyl group or a protected carboxyl group, $R^5$ is a carboxyl

group, a protected carboxyl group or a group of the formula: $-COO^-$, $R^6$ 15 a hydrogen atom or a lower alkyl group, $X^1$ is a reactive residue and the broken line means the presence or absence of a double bond, or a salt thereof.

**19.** A pharmaceutical composition which comprises an antimicrobially effective amount of the compound as claimed in any one of claims 1 to 12 or a pharmaceutically acceptable salt thereof, and a pharmaceutically acceptable carrier.

European Patent Office

# EUROPEAN SEARCH REPORT

Application Number

EP 91 31 1373

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | EP-A-0 289 002 (MEIJI SEIKA KAISHA)<br>* page 12 *<br>* page 36 - page 39; claims * | 1-19 | C07D501/20<br>C07D417/12<br>C07D215/24<br>A61K31/545 |
| A | EP-A-0 315 997 (MEIJA SEIKA KAISHA)<br>* page 1 - page 2 *<br>* page 19 - page 20; claims * | 1-19 | |
| A | WO-A-8 605 786 (MOCHIDA)<br>* page 142 - page 186; claims * | 1-19 | |
| A | CHEMICAL ABSTRACTS, vol. 110, no. 5, 30 January 1989, Columbus, Ohio, US;<br>abstract no. 38814W,<br>page 525 ;column L ;<br>* abstract *<br>& JP-A-6 351 388 (TEIJIN) | 1-19 | |
| A | EP-A-0 015 543 (HOECHST)<br>*Complete specification.* | 17 | TECHNICAL FIELDS SEARCHED (Int. Cl.5) |
| A | EP-A-0 240 015 (OTSUKA)<br>* page 78; example 27 *<br>* page 171 - page 172; claim 1 * | 16 | C07D |

The present search report has been drawn up for all claims

| Place of search | Date of completion of the search | Examiner |
|---|---|---|
| THE HAGUE | 30 JULY 1992 | LUYTEN H.W. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)